# EUROPEAN PATENT SPECIFICATION

(11) **EP 1 069 821 B1**
(45) Date of publication and mention of the grant of the patent: **08.06.2011**
(21) Application number: 99906799.4
(22) Date of filing: 08.02.1999
(51) Int. Cl.: A01N 1/02, A61K 35/12, C12P 19/34, C12N 15/85, C12N 15/86

(54) **METHOD OF CONTROLLING PROLIFERATION AND DIFFERENTIATION OF STEM AND PROGENITOR CELLS**
METHODE ZUR KONTROLLE DER PROLIFERATION UND DIFFERENZIERUNG VON STAMM- UND VORLÄUFERZELLEN
PROCEDE DE LUTTE CONTRE LA PROLIFERATION ET LA DIFFERENCIATION DE CELLULES SOUCHES ET PARENTES

(30) Priority: 17.02.1998 US 24195; 07.08.1998 US 130367
(43) Date of publication of application: 24.01.2001
(60) Divisional application: 03003275.9
(73) Proprietor: Gamida Cell Ltd., 95484 Jerusalem (IL); HADASIT MEDICAL RESEARCH SERVICES AND DEVELOPMENT LTD., 91120 Jerusalem (IL)
(72) Inventor: PELED, Tony, 90805 Mevaseret Zion (IL); FIBACH, Eitan, 90805 Mevaseret Zion (IL); TREVES, Avi, 90805 Mevaseret Zion (IL); FRIEDMAN, Mark, M., 43406 Raanana (IL)
(74) Representative: Jaenichen, Hans-Rainer
(86) International application number: PCT/US1999/002664
(87) International publication number: WO 1999/040783

(56) References cited:
- EP-A- 0 331 464
- WO-A-97/31647
- WO-A-97/33978
- WO-A1-00/18885
- US-A- 5 631 219
- US-A1- 2002 159 981
- CICUTTINI F M ET AL: "Support of human cord blood progenitor cells on human stromal cell lines transformed by SV40 large T antigen under the influence of an inducible (metallothionein) promoter" BLOOD, W.B. SAUNDERS, PHILADELPHIA, VA, US, vol. 80, no. 1, 1 July 1992 (1992-07-01), pages 102-112, XP002052872 ISSN: 0006-4971
- PERCIVAL SS ET AL: "HL-60 cells can be made copper deficient by incubating with tetraethylenepentamine" J. NUTRITION, vol. 122, 1992, pages 2424-2429, XP001012729
- MORIMOTO S ET AL: "EDTA induces differentiation and suppresses proliferation of promyelocytic leukemia cell line HL-60 - possible participation of zinc- " BIOCHEMISTRY INTERNATIONAL, vol. 28, no. 2, October 1992 (1992-10), pages 313-321, XP001021407
- PERROTTI D ET AL: "Overexpression of the zinc finger protein MZF1 inhibits hematopoietic development from embryonic stem cells: correlation with negative regulation of CD34 and C-MYB promoter activity" MOLECULAR AND CELLULAR BIOLOGY, WASHINGTON, DC, US, vol. 15, no. 11, 1 November 1995 (1995-11-01), pages 6075-6087, XP002053235 ISSN: 0270-7306
- MURRAY MJ ET AL: "Modulation of murine lymphocyte and macrophage proliferation by parenteral zinc" CLIN EXP IMMUNOL, vol. 53, 1983, pages 744-749, XP001012814
- REID et al., "Interactions of Tumor Necrosis Factor with Granulocyte-Macrophage Colony-Stimulating Factor and Other Cytokines in the Regulation of Dendritic Cell Growth In Vitro from Early Bipotent CD34+ Progenitors in Human Bone Marrow", J. IMMUNOLOGY, 15 October 1992, Vol. 149, No. 8, pages 2681-2688, XP002918856
- NARITA et al., "Cardiomycyte Differentiation by GATA-4-Deficient Embryonic Stem Cells", DEVELOPMENT, 1996, Vol. 122, No. 19, pages 3755-3764, XP002918857
- HAMMOND W P ET AL: "Suppression of in vitro granulocytopoiesis by captopril and penicillamine" EXP. HEMATOL., vol. 16, 1988, pages 674-680,
- SEKHAR M ET AL: "Retroviral transduction of CD34-enriched hematopoietic progenitor cells under serum-free conditions" HUMAN GENE THERAPY, vol. 7, 1 January 1996 (1996-01-01), pages 33-38,
- PIACIBELLO W ET AL: "Extensive amplification and self-renewal of human primitive hematopoietic stem cells from cord blood" BLOOD, vol. 89, no. 8, 15 April 1997 (1997-04-15), pages 2644-2653,
- HAMMOND W P ET AL: "Suppression of in vitro granulocytopoiesis by captopril and penicillamine" EXP. HEMATOL., vol. 16, 1988, pages 674-680,
- PERCIVAL S: "Copper and immunity" AMERICAN JOURNAL OF CLINICAL NUTRITION, vol. 67(suppl.), 1998, pages 1064S-1068S,
- CHISI J ET AL: "Inhibitory action of the peptide AcSDKP on the proliferative state of hematopoietic stem cells in the presence of Captopril but not Lisinopril" STEM CELLS, vol. 15, 1997, pages 455-460,

## Description

### FIELD AND BACKGROUND OF THE INVENTION

The present invention relates to the embodiments characterized in the claims. Thus, it relates to
(1) a method of expanding hematopoietic stem and progenitor cells *ex-vivo,* while at the same time inhibiting differentiation of said hematopoietic stem and progenitor cells, the method comprising providing said hematopoietic stem and progenitor cells *ex-vivo* with conditions for cell proliferation and with a transition metal chelator selected from the group consisting of tetraethylenepentamine (TEPA), ethylenediamine (EDA), pentaethylenehexamine (PEHA), triethylenetetramine (TETA), penicilamine and captopril, wherein said conditions for cell proliferation comprise nutrients and at least an early-acting cytokine or early-acting cytokines, wherein said hematopoietic stem and progenitor cells are therapeutic hematopoietic stem and progenitor cells, and wherein said chelator and said proliferation conditions result in
   (i) prolonged active proliferation;
   (ii) long-term expansion of clonogenic cells (CFUc); and
   (iii) maintenance of undifferentiated cells in their undifferentiated state;
      with the proviso that said hematopoietic cells are not human embryonic stem cells obtained from a human embryo;
(2) a method of transducing expanded, undifferentiated therapeutic hematopoietic stem and progenitor cells with an exogene, the method comprising:
   (a) expanding and inhibiting differentiation of said hematopoietic stem and progenitor cells by:
      (i) providing said hematopoietic stem and progenitor cells with conditions for cell proliferation, wherein said conditions for cell proliferation comprise nutrients and at least an early acting cytokine or early-acting cytokines and wherein said hematopoietic stem and progenitor cells are therapeutic hematopoietic stem and progenitor cells;
      (ii) contacting said hematopoietic stem and progenitor cells with a transition metal chelator selected from the group consisting of TEPA, EDA, PEHA, TETA, penicilamine and captopril, wherein said chelator and said proliferation conditions result in prolonged active proliferation; long-term expansion of clonogenic cells (CFUc); and maintenance of undifferentiated cells in their undifferentiated state; thereby inhibiting differentiation and expanding said hematopoietic cells *ex-vivo;* and
   (b) transducing said therapeutic expanded, undifferentiated hematopoietic stem and progenitor cells with the exogene, with the proviso that said hematopoietic stem and progenitor cells are not human embryonic stem cells obtained from a human embryo;
(3) a therapeutic ex vivo cultured hematopoietic stem and progenitor cell population comprising undifferentiated hematopoietic stem and progenitor cells expanded according to the methods of any of claims 1-14, said population having:
   (i) a greater proportion of proliferating hematopoietic stem and progenitor cells;
   (ii) a greater proportion of clonogenic cells (CFUc); and
   (iii) a greater proportion of undifferentiated cells maintained in their undifferentiated state
      as compared to a similar population of hematopoietic stem and progenitor cells ex-vivo cultured without contacting said transition metal chelator;
(4) a method of preservation of undifferentiated hematopoietic stem and progenitor cells comprising contacting the undifferentiated hematopoietic stem and progenitor cells with a transition metal chelator selected from the group consisting of TEPA, EDA, PEHA, TETA, penicilamine and captopril, wherein said contacting is performed in at least one of the steps of harvesting, isolating and storage of the undifferentiated hematopoietic cells, with the proviso that said hematopoietic cells are not human embryonic stem cells obtained from a human embryo;
(5) stem cell collection bags, supplemented with an amount of a transition metal chelator selected from the group consisting of TEPA, EDA, PEHA, TETA, penicilamine and captopril, said amount sufficient to inhibit differentiation of a population of undifferentiated hematopoietic cells;
(6) the use of a therapeutic hematopoietic cell composition comprising a population of undifferentiated hematopoietic stem and progenitor cells expanded according to the methods of any of claims 1-14 for the preparation of a medicament for treating a subject in need of hematopoietic stem cell transplantation, wherein said population is characterized by having:
   (i) a greater proportion of proliferating hematopoietic stem and progenitor cells;
   (ii) a greater proportion of clonogenic cells (CFUc); and
   (iii) a greater proportion of undifferentiated cells maintained in their undifferentiated state
      as compared to a similar population of hematopoietic stem and progenitor cells ex-vivo cultured without contacting said transition metal chelator;
(7) a therapeutic ex vivo cultured hematopoietic stem and progenitor cell population comprising undifferentiated hematopoietic stem and progenitor cells expanded according to the methods of any of claims 1-14, wherein said cell population is provided in a medium comprising said transition metal chelator.

### Cell differentiation and proliferation

Normal production of blood cells (hematopoiesis) involves the processes of proliferation and differentiation which are tightly coupled. In most hematopoietic cells following division the daughter cells undergo a series of progressive changes which eventually culminate in fully differentiated (mature), functional blood cells, which in most part are devoid of proliferative potential.

Thus, the process of differentiation limits, and eventually halts cell division. Only in a small minority of the hematopoietic cells, known as stem cells, cell division may result in progeny which are similar or identical to their parental cells. This type of cell division, known as self-renewal, is an inherent property of stem cells and helps to maintain a small pool of stem cells in their most undifferentiated state. Some stem cells lose their self-renewal capacity and following cell division differentiate into various types of lineage committed progenitors which finally give rise to mature cells. While the latter provide the functional capacity of the blood cell system, the stem cells are responsible for the maintaining of hemopoiesis throughout life despite a continuous loss of the more differentiated cells through apoptosis (programmed cell death) and/or active removal of aging mature cells by the reticuloendothelial system.

As further detailed below, expansion of the stem cell and other defined lympho-hemopoietic cell subpopulations by *ex vivo* culturing could have important clinical applications.

A variety of protocols have been suggested and experimented for enrichment of such populations. The main experimental strategies employed include incubation of mononuclear cells with or without selection of CD₃₄⁺ (8); with different cocktails of early and late growth factors (17); with or without serum (7); in stationary cultures, rapid medium exchanged cultures (18) or under continuous perfusion (bioreactors) (6); and with or without established stromal cell layer (19).

Although a significant expansion of intermediate and late progenitors was often obtained during 7-14 days *ex vivo* cultures, the magnitude of early hematopoietic (CD₃₄⁺CD₃₈⁻) stem cells with high proliferative potential, usually declined (6, 20-22).

Thus, these cultures do not result in true stem cell expansion, but rather in proliferation and differentiation of the stem cells into pre-progenitor cells, accompanied by depletion of the primitive stem cell pool.

In order to achieve maximal *ex vivo* expansion of stem cells the following condition should be fulfilled: (i) differentiation should be reversibly inhibited or delayed and (ii) self-renewal should be maximally prolonged.

### Role of Copper in cell differentiation:

The possible involvement of Copper in hemopoietic cell development could be inferred from the following findings:

***Clinical symptoms in Copper deficiency:*** Copper deficiency can result from hereditary defects, such as Menkes syndrome or Celiac disease, or from acquired conditions. The latter is typically associated with malnourishment. It may be caused by Copper non-supplemented total parenteral nutrition (e.g., following intestinal resection), by consumption of high levels of Zinc, which interferes with Copper utilization, in underweight and/or cow milk (poor source of Copper) fed newborns, which may result in severe cases in Shwanchman syndrome. Unbalanced treatment with Copper chelators in Copper overload cases such as in Wilson's disease may also lead to Copper deficiency.

The clinical symptoms of Copper deficiency may include impairment of growth, brain development, bone strength and morphology, myocardial contractility, cholesterol and glucose metabolism, host defence (immune) mechanisms and more.

Of particular relevance to this study is the fact that Copper deficiency is often associated with hematological abnormalities, including anemia, neutropenia and thrombocytopenia. All these pathological manifestations are unresponsive to iron therapy, but are rapidly reversed following Copper supplementation (27-28).

The mechanism by which Copper deficiency leads to neutropenia is unknown. Among the possible causes, either alone or in combination, are: (i) early death of progenitor cells in the bone marrow (BM); (ii) impaired formation of neutrophils from progenitor cells in the BM; (iii) decrease in cellular maturation rate in the BM; (iv) impaired release of neutrophils from the BM to the circulation; (v) enhanced elimination rate of circulating neutrophils.

Examination of the BM of neutropenic Copper-deficient patients demonstrates the absence of mature cells ("maturation arrest"). It has been shown that cells derived from such BM did not form colonies in semi-solid medium containing Copper deficient serum, but retained the potential for normal colony growth in Copper containing serum. These results indicate the presence of intact progenitors in the patient's BM, and suggest that the block in development occurs distal to the progenitor stage (29-30).

***The effect of Copper in cell lines:*** The effect of Copper was also studied *in vitro* established cell lines (31-34). One such line (HL-60) was derived from a patient with acute promyelocytic leukemia. These cells, that have the characteristics of myeloblasts and promyelocytes, can grow indefinitely in culture. Upon addition of various agents, such as retinoic acid (RA), to the culture medium, the cells undergo differentiation, which results in cells which demonstrate some, but not all, features of mature granulocytes.

The study of Copper status in these cells has shown that although the cytosolic Copper content per cell was not significantly different in RA-treated cells compared to untreated cells, the Copper content per protein content was doubled. This is due to the fact that RA-treated cells have about half the protein content as compared to their untreated counterpart. Using ⁶⁷Cu, it has been shown that the rate of Copper uptake was significantly faster during the two first days of RA treatment, but not at later times. The intracellular distribution of ⁶⁷Cu was found predominantly in high molecular weight (MW) fractions (> 100 kD) and a lower MW fraction of about 20 kD, with a higher proportion of Copper present in the high MW fractions in RA-treated cells.

Addition of excess Copper to regular serum-supplemented growth medium modestly increased RA-induced differentiation. Although RA-treated HL-60 cells do not necessarily represent normal cell development, these results point to the possibility that neutrophilic differentiation may require Copper.

In other experiments it has been shown that HL-60 cells can be made Copper deficient by treatment with Copper chelators, and that following such treatment their viability and growth rate were unaffected.

Although all these phenomena have been attributed to Copper, it has been reported that some clinical and biological effects are shared by Copper and other transition metals:

For example, clinical symptoms similar to those observed in Copper-deficiency could also be observed following consumption of high levels of Zinc (40-42), which has been known to interfere with Copper utilization (e.g., 43).

In a study of human hepatocellular carcinoma it was found that the concentrations of both Copper and Zinc in the tumor tissue decreased with the degree of histological differentiation (44).

In another study it was shown that addition of Copper, Zinc and Ferrum to primary cultures of rat hepatocytes induced cell replication and formation of duct-like structures. The cells lining the ducts became morphologically and biochemically characteristic of bile duct cells (45).

Various transition metals are known to influence the production and activities of many enzymes and transcription factors associated with differentiation. Examples include the Cu/Zn containing superoxide dismutase (46); the metallothioneins and their transcription regulating factors (e.g., MTF-1) (47-49); the 70 kDa heat shock protein (hsp70) (50); the p62 protein which associates with the ras-GTPase activating protein during keratinocyte differentiation (51); a neutral sphingomyelinase which is activated during induced differentiation of HL-60 cells (52); and the bovine lens leucine aminopeptidase (53).

While reducing the present invention to practice, it was found that a series of chemical agents that bind (chelate) transition metals, Copper in particular, can inhibit (delay) the process of differentiation of stem cells as well as intermediate and late progenitor cells and thereby stimulate and prolong the phase of active cell proliferation *ex vivo.* This newly discovered effect of Copper and other transition metals depletion (either partial or complete depletion) was used for maximizing the *ex vivo* expansion of various types of hemopoietic cells.

### SUMMARY OF THE INVENTION

According to the present invention there is provided a method of expanding a population of cells, while at the same time inhibiting differentiation of the cells as further characterized in the claims.

According to still further features in the described preferred embodiments the early acting cytokines are selected from the group consisting of stem cell factor, FLT3 ligand, interleukin-6, thrombopoietin and interleukin-3.

According to still further features in the described preferred embodiments the method further comprises providing a late acting cytokine or late acting cytokines.

According to still further features in the described preferred embodiments the late acting cytokines are selected from the group consisting of granulocyte colony stimulating factor, granulocyte/macrophage colony stimulating factor and erythropoietin.

According to still further features in the described preferred embodiments the cells are derived from a source selected from the group consisting of bone marrow, peripheral blood and neonatal umbilical cord blood.

According to still further features in the described preferred embodiments the cells are enriched for hemopoietic CD₃₄+ cells.

The cells may be selected from the group consisting of non-differentiated stem cells and committed progenitor cells.

According to further features in preferred embodiments of the invention described below, there is provided a method of transducing expanded, undifferentiated therapeutic hematopoietic stem and progenitor cells with an exogene as further characterized in the claims.

According to still further features in the described preferred embodiments transducing is effected by a vector including the exogene.

According to further features in preferred embodiments of the invention described below, there is provided a therapeutical *ex vivo* cultured hematopoietic stem and progenitor cell population as further characterized in the claims. According to another embodiment of the present invention there is provided a method of preservation of undifferentiated hematopoietic stem and progenitor cells as further characterized in the claims.
Respectively, further according to the present invention there are provided stem cells collection bags as further characterized in the claims.

The present invention successfully addresses the shortcomings of the presently known configurations by providing a method of propagating cells, yet delaying their differentiation by transition metals deficiency.

Additional features and advantages of the method according to the present invention are described hereinunder.

### BRIEF DESCRIPTION OF THE DRAWINGS

The invention herein described, by way of example only, with reference to the accompanying drawings, wherein:

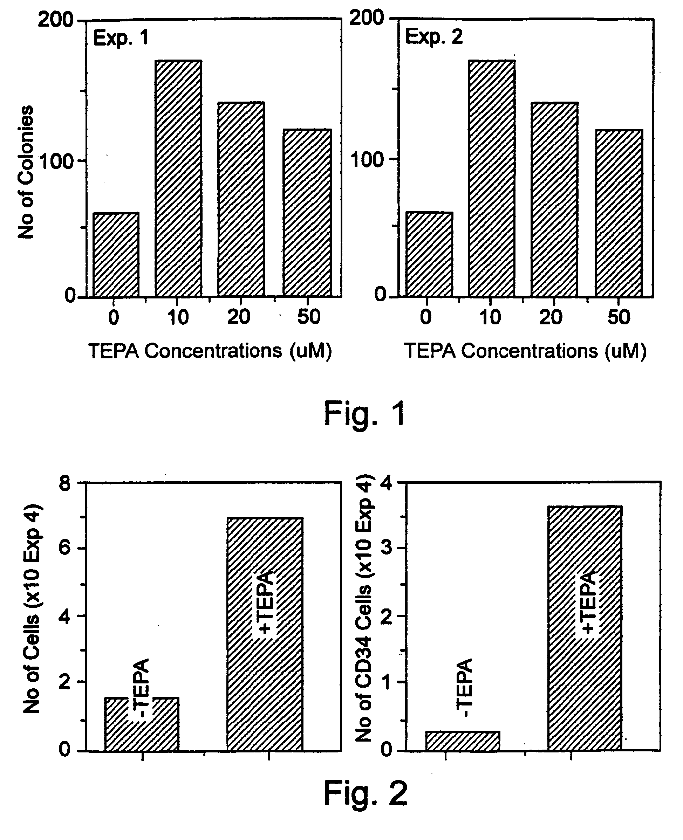

FIG. 1 shows the short-term effect of TEPA on the clonlogenic potential of CD₃₄ cells. Cord blood-derived CD₃₄ cells were plated in liquid culture, at 3 x 10⁴ cell/ml, in the presence of low dose cytokines: FLT3 ligand - 5ng/ml, SCF - 10ng/ml, IL-6 - 10ng/ml, with or without different concentrations of TEPA. On day 7, aliquots of 0.1 ml were assayed for colony forming cells by cloning the cells in semi-solid medium and scoring colonies after 14 days. Results of two independent experiments are presented.
FIG. 2 shows the short-term effect of TEPA on total and CD₃₄ cells. Cord blood-derived CD₃₄ cells were plated in liquid culture in the presence of FLT 3 ligand - 5ng/ml, SCF - 10ng/ml, IL-6 - 10ng/ml, with or without of TEPA (20µM). On day 7, the wells were demi-depopulated by removal of one half the culture volume and replacing it with fresh medium and IL-3 (20ng/ml). On day 14, the percentage of CD₃₄ cells (right) and the total cell number (left) multiplied by the dilution factor were determined.

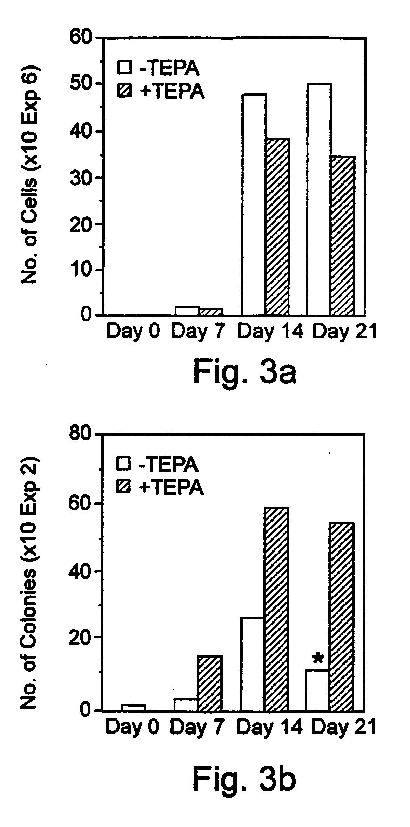

FIG. 3 shows the long-term effect of TEPA on cell number and clonogenic potential of CD₃₄ cells. Cord blood-derived CD₃₄ cells were plated in liquid culture, at 3 x 10⁴ cells/ml, in the presence of high dose cytokines: FLT 3 ligand - 50g/ml, SCF - 50ng/ml, IL-6 - 50ng/ml, IL-3 - 20ng/ml, G-CSF - 10ng/ml, EPO - 1U/ml, with or without TEPA (20µM). On day 4, the cultures were diluted 1:10 with 0.9 ml fresh medium supplemented with cytokines and TEPA. On day 7, 14 and 21, the cultures were demi-depopulated by removal of one half the culture volume and replacing it with fresh medium, cytokines and TEPA, as indicated. Cells of the harvested medium were count and aliquots equivalent to 1 x 10³ initiating cells were cloned in semi-solid medium. The numbers of cells (up) in the liquid culture and of colonies (down) in the semi-solid culture, multiplied by the dilution factors, are represented. * denotes small colonies and cell clusters.

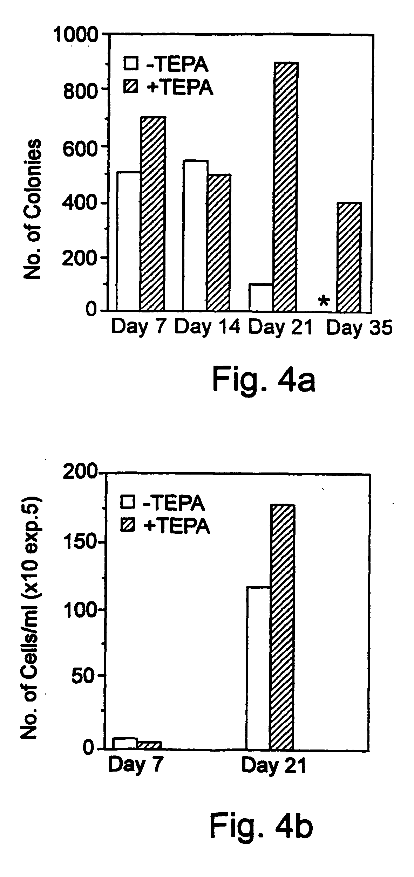

FIG. 4 shows the long-term effect of TEPA on CD₃₄ cells cultured with early cytokines. Cord blood-derived CD₃₄ cells were plated in liquid culture in the presence of: FLT 3 ligand - 50ng/ml, SCF - 50ng/ml and thrombopoietin (TPO) - 20ng/ml, with or without TEPA (10µM). At weekly intervals, the cultures were demi-depopulated by removal of one half the culture volume and replacing it with fresh medium, cytokines and TEPA, as indicated Cells of the harvested medium were count and aliquots equivalent to 1 x 10³ initiating cells were cloned in semi-solid medium. The numbers of cells (down) in the liquid culture and of colonies (up) in the semi-solid culture, multiplied by the dilution factors, are represented. * denotes that no colonies developed.

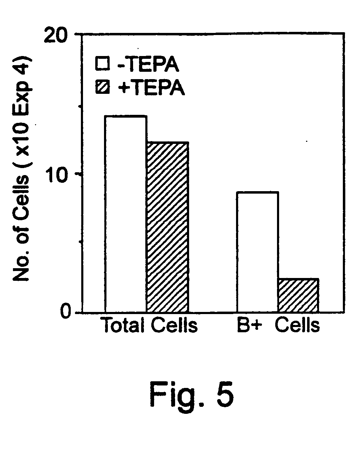

FIG. 5 shows the effect of TEPA on development of erythroid precursors. Peripheral blood mononuclear cells, obtained from an adult normal donor, were cultured in the erythroid two-phase liquid culture system (23-25). The second phase of the culture was supplemented either without or with 10 µM of TEPA. Cultures were analyze for total cells and hemoglobin-containing [benzidine positive (B⁺)] cells after 14 days.

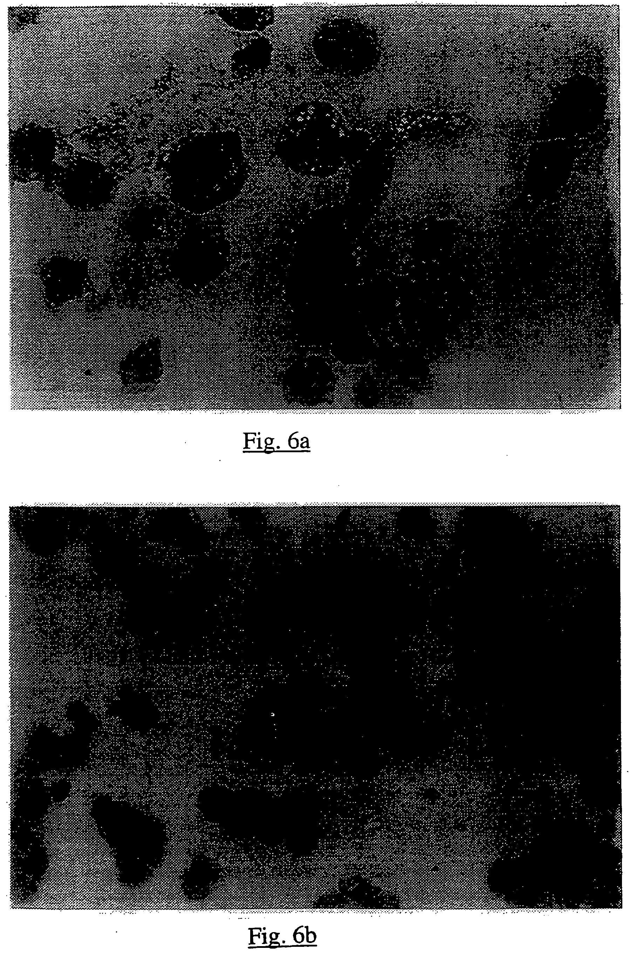

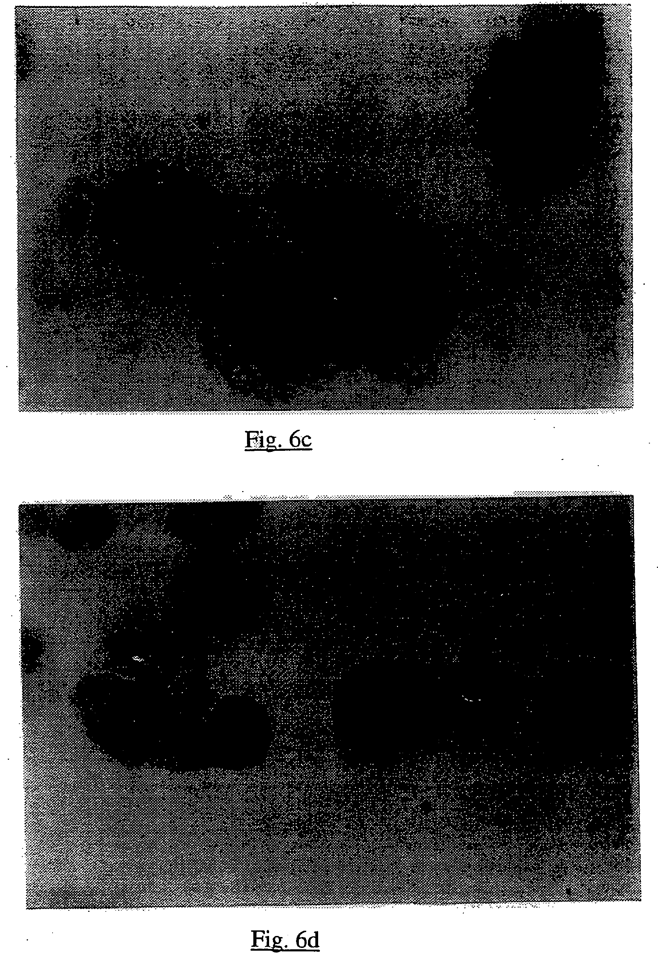

FIGs. 6a-d show the effect of TEPA on cell maturation. Morphology of cells in long-term (7 weeks) cultures in the absence (6a and 6c) and presence (6b and 6d) of TEPA is shown. Cytospin prepared slides were stained with May-Grunwald Giemsa. Magnifications: 6a and 6b x 600; 6c and 6d x 1485.

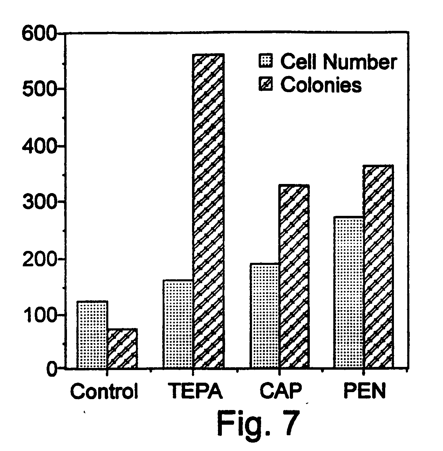

FIG. 7 shows the effect of transition metal chelators on cell number and clonogenic of CD₃₄ cells initiated cultures. Cord blood-derived CD₃₄ cells were plated in liquid cultures in the presence of FLT 3 ligand (-20ng/ml, SCF - 20ng/ml, IL-3 - 20ng/ml, IL-6 - 20ng/ml, and either TEPA - 10 µM, captopril (CAP) - 10 µM or Penicillamine (PEN) - 10 µM, as indicated. On day 7, cells were counted and culture aliquots equivalent to 1 x 10³ initiating cells were plated in semi-solid medium. The bars present the total cell number (x10³/ml) on day 7 and the number of colonies per plate 14 days following cloning.

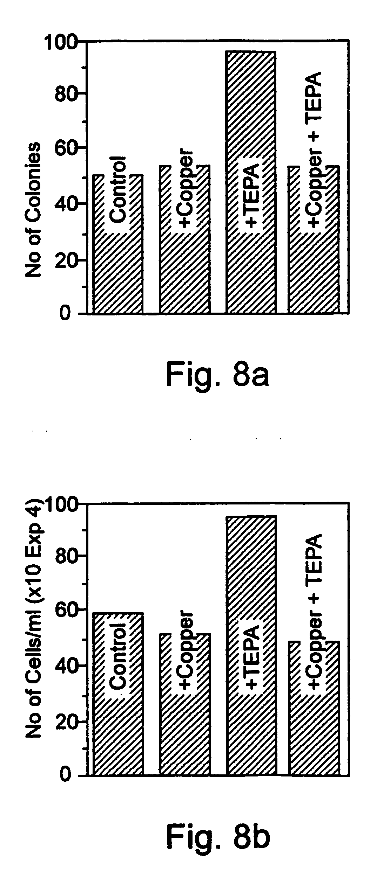

FIG. 8 shows the effect of Copper on the clonogenic potential and total cell number of CD₃₄ cells. Cord blood-derived CD₃₄ cells were plated in liquid cultures in the presence of cytokines: FLT 3 ligand - 10ng/ml, SCF - 10ng/ml, IL-3 - 10ng/ml, IL-6 - 10ng/ml. Cultures were supplemented with Copper-sulfate - 5µ M and TEPA - 20µM, as indicated. On day 7, cells were counted (down) and aliquots equivalent to 1 x 10³ initiating cells were plated in semi-solid medium. Colonies were scored after 14 days (up).

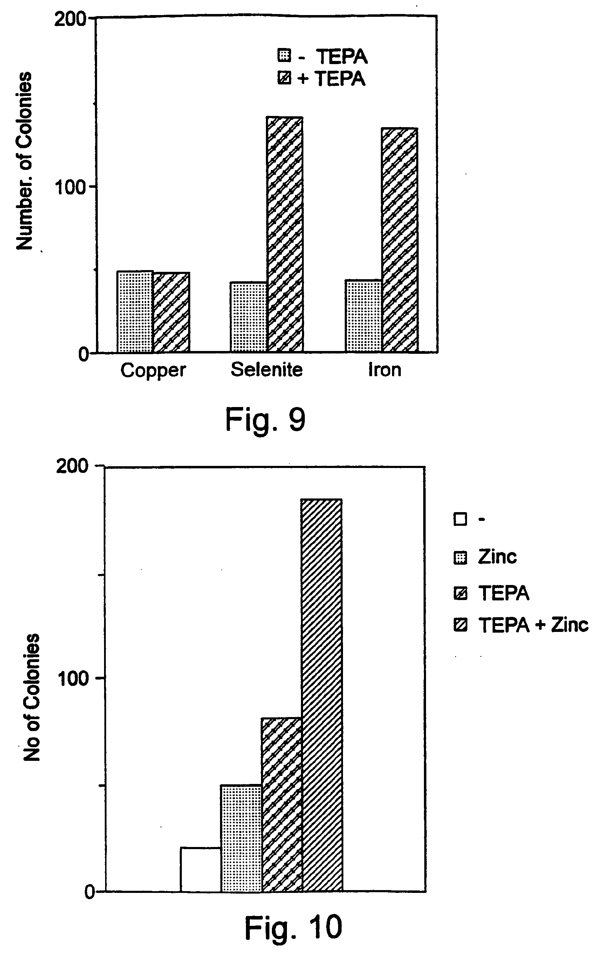

FIG. 9 shows the effect of ions on the clonogenic potential of cultured CD₃₄ cells. Cord blood-derived CD₃₄ cells were plated in liquid cultures in the presence of FLT 3 ligand - 10ng/ml, SCF - 10ng/ml, IL-3 - 10ng/ml, IL-6 - 10ng/ml, and either with or without TEPA - 10 µM. The cultures were supplemented with Copper-sulfate - 5mM, sodium selenite - 5mM or iron-saturated transferrin 0.3 mg/ml, as indicated. On day 7, culture aliquots equivalent to 1 x 10³ initiating cells were plated in semi-solid medium. Colonies were scored after 14 days.
FIG. 10 shows the effect of Zinc on the proliferative potential of CD₃₄ cells. Cord blood-derived CD₃₄ cells were plated in liquid cultures in the presence of FLT 3 ligand - 10ng/ml, SCF - 10ng/ml, IL-3 - 10ng/ml, IL-6 - 10ng/ml, and either TEPA - 10 µM or Zinc-sulfate - 5mM or both. On day 7, aliquots equivalent to 1 x 10³ initiating cells were plated in semi-solid medium. Colonies were scored after 14 days.

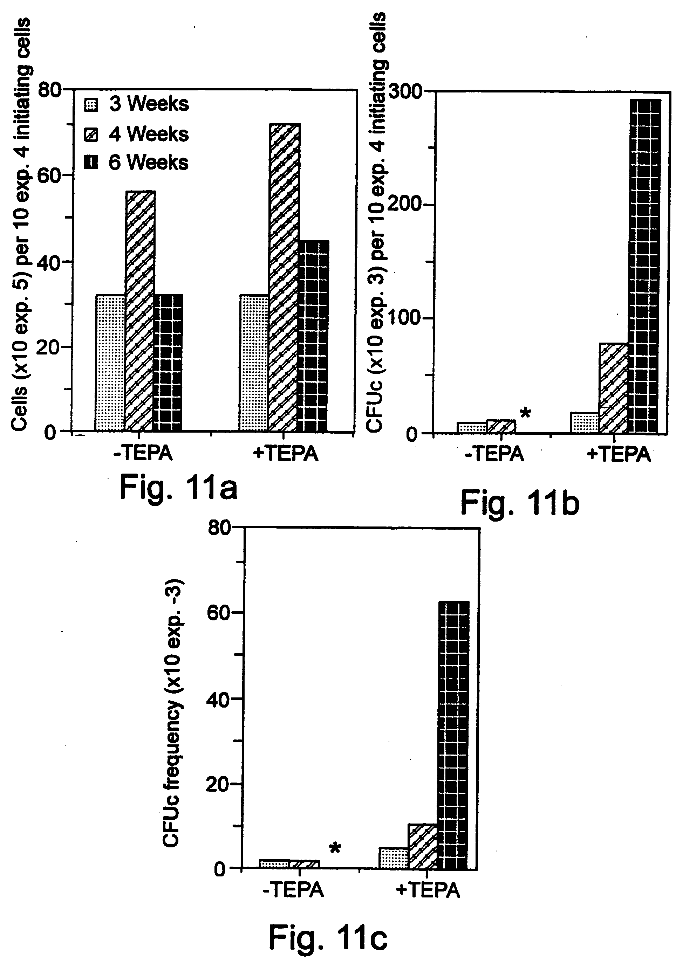

FIGs. 11a-c show the effect of TEPA on long-term CD₃₄ cultures. Cultures were initiated with 10⁴ cord blood-derived CD₃₄ cells by plating purified cells in liquid medium in the presence of SCF, FLT 3 ligand and IL-6 (50ng/ml each) and IL-3 (20ng/ml) with or without TEPA (10µM). At weekly intervals, the cultures were demi-depopulated by removal of half the cells followed by addition of fresh medium, cytokines and TEPA. At the indicated weeks, cells were counted and assayed for colony forming cells (CFUc) by cloning in semi-solid medium. CFUc frequency was calculated as number of CFUc per number of cells. Cloning of purified CD₃₄ cells on day 1 yielded 2.5x10³ CFUc per 10⁴ initiating cells. * denotes that no colonies developed.

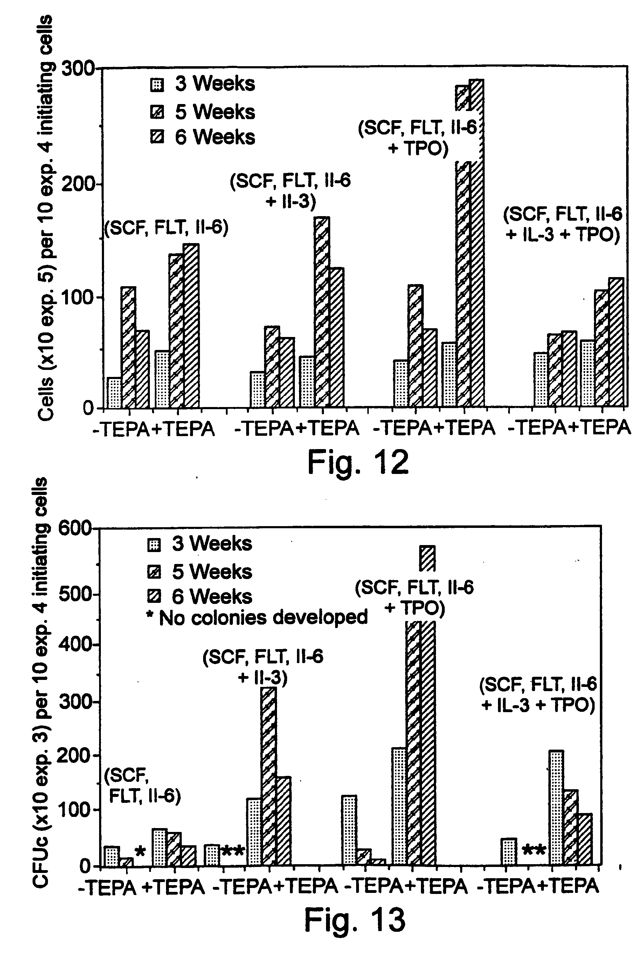

FIGs. 12-14 show the effect of TEPA on cell proliferation, CFUc and CFUc frequency in the presence of different combination of early cytokines. Cord blood-derived CD₃₄ cells were cultured as detailed in Figures 11a-c in liquid medium in the presence of SCF, FLT 3 ligand and IL-6 (SCF, FLT, II-6), each at 50ng/ml, with or without TEPA (10µM). In addition, cultures were supplemented with either IL- 3 (20ng/ml), TPO (50ng/ml) or both, as indicated. At weekly intervals, the cultures were demi-depopulated and supplemented with fresh medium, cytokines and TEPA. At the indicated weeks, the cells were counted (Figure 12), assayed for CFUc (Figure 13) and the CFUc frequency calculated (Figure 4). * denotes that no colonies developed.

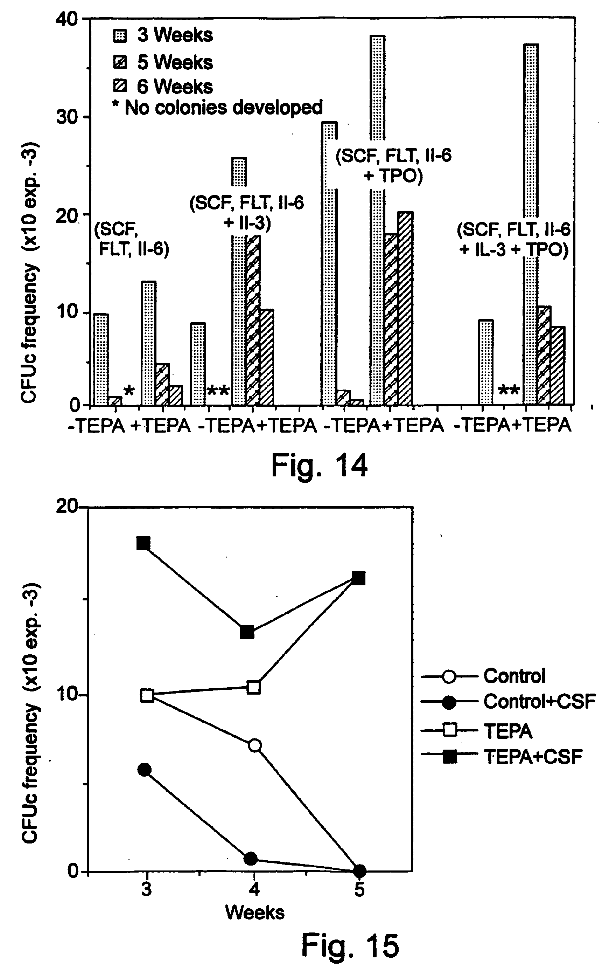

FIG. 15 shows the effect of G-CSF and GM-CSF on CFUc frequency of control and TEPA-supplemented CD₃₄ cultures. Cord blood-derived CD₃₄ cells were cultured as detailed in Figures 11a-c. After one week, half of the control and TEPA cultures were supplemented with the late-acting cytokines G-CSF and GM-CSF (10ng/ml each). At weekly intervals, the cultures were demi-depopulated and supplemented with fresh medium, cytokines and TEPA. At weeks 3, 4 and 5, cells were counted, assayed for CFUc and CFUc frequency calculated.

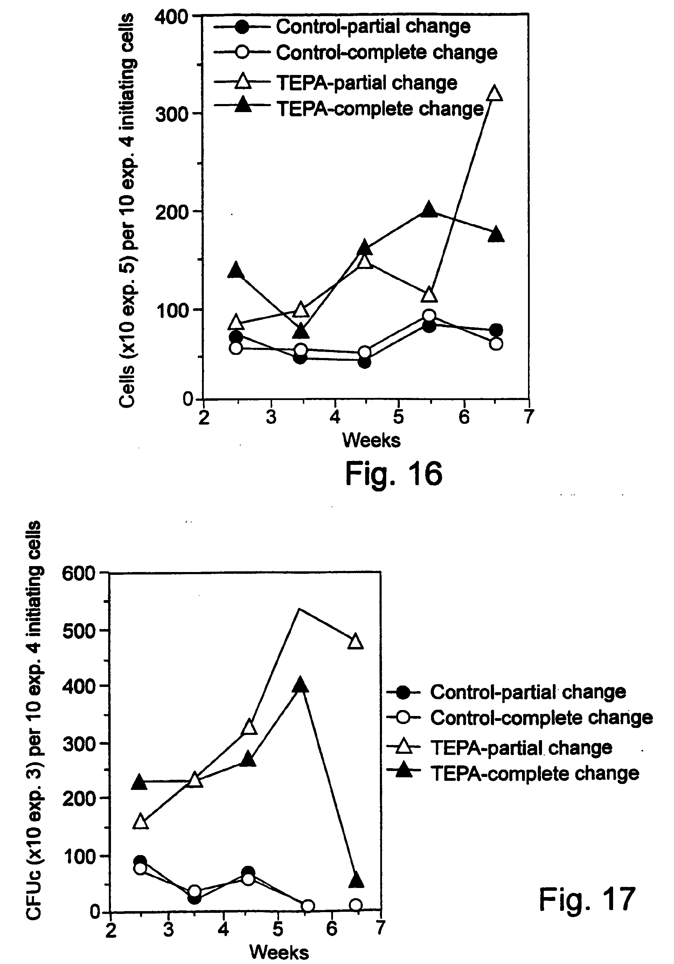

FIGs. 16-17 show the effect of partial or complete medium + TEPA change on long-term cell proliferation and CFUc production. Cord blood-derived CD₃₄ cells were cultured as detailed in Figures 11a-c. At weekly intervals, the cultures were demi-depopulated and supplemented with fresh medium, cytokines and TEPA. At weekly intervals, half of the culture content (cells and supernatant) was removed and replaced by fresh medium, cytokines with or without TEPA (partial change). Alternatively, the whole content of the culture was harvested, centrifuged, the supernatant and half of the cells discarded and the remaining cells recultured in fresh medium, cytokines with or without TEPA (complete change). At the indicated weeks the number of cells (Figure 16) and CFUc (Figure 17) were determined.

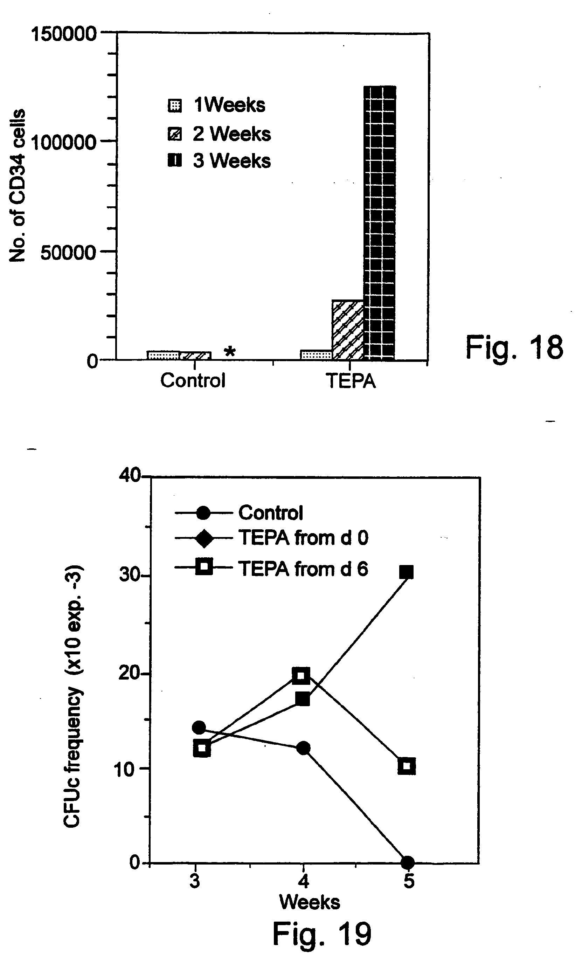

FIG. 18 show the effect of TEPA on CD₃₄ cell expansion. Cord blood-derived CD₃₄ cells were cultured as detailed in Figures 11a-c. At weeks 1, 2 and 3, CD₃₄⁺ cells were enumerated by flow cytometry. * denotes that no colonies developed.
FIG. 19 shows the effect of delayed addition of TEPA on CFUc frequency. Cord blood-derived CD₃₄ cells were cultured as detailed in Figures 11a-c. TEPA (10µM was added at the initiation of the cultures (day 1) or 6 days later. At weekly intervals, the cultures were demi-depopulated and supplemented with fresh medium, cytokines and TEPA. At weeks 3, 4 and 5, cells were counted, assayed for CFUc and the CFUc frequency was calculated.

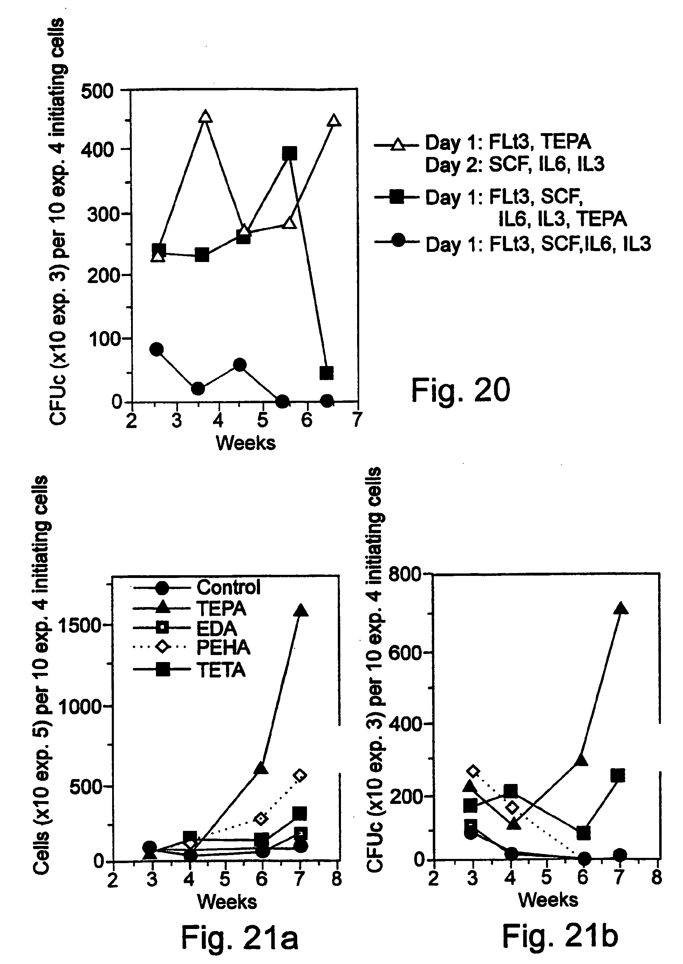

FIG. 20 show the effect of short-term preincubation with a single cytokine on long-term CFUc production. Cord blood-derived CD₃₄ cells were cultured as detailed in Figures 11a-c. Cultures were supplemented on day 1 with or without TEPA (10 µM) and with SCF, FLT 3 ligand IL-6, (50ng/ml each) and IL-3 (20ng/ml). Alternatively, cultures were supplemented on day 1 with TEPA (10 µM) and FLT 3 ligand (50 ng/ml) as a single cytokine. SCF, IL-6 (50ng/ml each) and IL-3 (20ng/ml) were added to these cultures at day2. At weekly intervals, the cultures were demi-depopulated and supplemented with fresh medium, cytokines and TEPA. At the indicated weeks cells were assayed for CFUc.
FIGs. 21a-b show the effect of polyamine chelating agents on CD₃₄ cell cultures. Cord blood-derived CD₃₄ cells were cultured as detailed in Figures 11a-c. The polyamine chelating agents tetraethylenepentamine (TEPA), pentaethylenehexamine (PEHA), ethylenediamine (EDA) or triethylene-tetramine (TETA) were added, at different concentrations. At weekly intervals, the cultures were demi-depopulated and supplemented with fresh medium, cytokines and chelators. At weeks 3, 4, 6 and 7, cells were counted and assayed for CFUc. The results presented are for concentrations with optimal activity: TEPA - 40 µM, PEHA - 40 µM, EDA - 20 µM and TETA - 20µM.

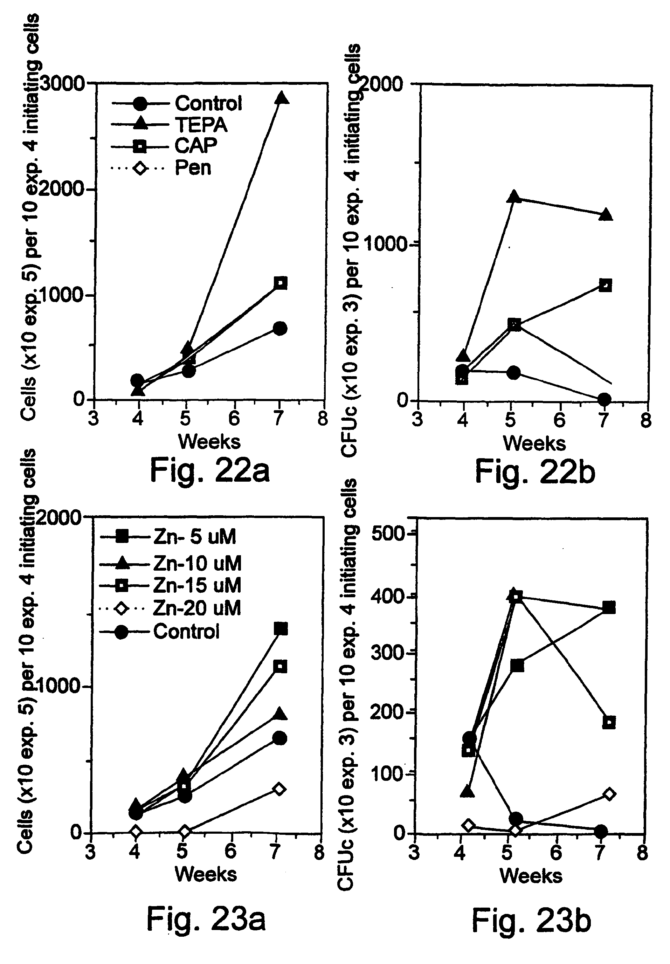

FIGs. 22a-b show the effect of transition metal chelating agents on CD₃₄ cell cultures. Cord blood-derived CD₃₄ cells were cultured as detailed in Figures 11a-c. The chelators Captopril (CAP), Penicilamine (PEN) and TEPA were added, at different concentrations. At weekly intervals, the cultures were demi-depopulated and supplemented with fresh medium, cytokines and chelators. At the weeks 4, 5 and 7, cells were counted and assayed for CFUc. The results presented are for concentrations with optimal activity: TEPA - 10 µM, PEN - 5 µ M and CAP - 40 µM.
FIGs. 23a-b show the effect of Zinc on CD₃₄ cell cultures. Cord blood-derived CD₃₄ cells were cultured as detailed in Figures 11a-c. Zinc (Zn) was added, at different concentrations, on day 1. At weekly intervals, the cultures were demi-depopulated and supplemented with fresh medium, cytokines and Zn. At the weeks 4, 5 and 7, cells were counted and assayed for CFUc.

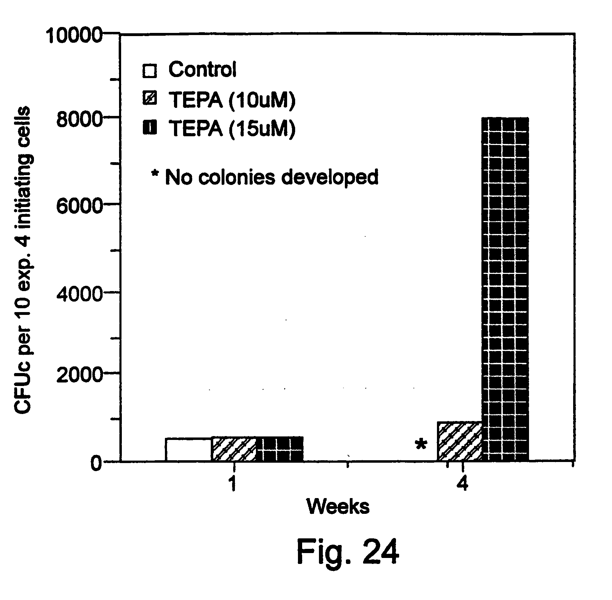

FIG. 24 shows the effect of TEPA on peripheral blood derived CD₃₄ cell cultures. Peripheral blood-derived CD₃₄ cells were cultured as detailed in Figures 11a-c. Cultures were supplemented with or without TEPA. At weekly intervals, the cultures were demi-depopulated and supplemented with fresh medium and TEPA. At weeks 1 and 4, and, cells were assayed for CFUc. * denotes that no colonies developed.

### DESCRIPTION OF THE PREFERRED EMBODIMENTS

The present invention is of a method of controlling proliferation and differentiation of hematopoietic stem and progenitor cells as further characterized in the claims which can be used to provide a therapeutical *ex vivo* cultured cell preparation which includes a large population of cells, in which differentiation was inhibited while expansion propagated. Specifically, the present invention can be used to provide hematopoietic stem cells, as well as progenitor cells, for hematopoietic cell transplantations, stem cells suitable for generic manipulations, which may be used for gene therapy, and new treatment means for diseases, such as, but not limited to, β-hemoglobinopathia.

The present invention relates to a method of controlling proliferation and differentiation of hematopoietic stem and progenitor cells as further characterized in the claims. More particularly, the present invention relates to a method of imposing proliferation yet restricting differentiation of hematopoietic stem and progenitor cells by modifying the availability of transition metals, Copper in particular, as further characterized in the claims.

The principles and operation of a method according to the present invention may be better understood with reference to the drawings and accompanying descriptions and examples.

In the course of the present study it was found that a series of chemical agents that bind (chelate) Copper and other transition metals, or that interfere with Copper metabolism can reversibly inhibit (delay) the process of differentiation of stem cells as well as intermediate and late progenitor cells and thereby stimulate and prolong the phase of active cell proliferation.

This newly discovered effect of transition metal depletion was utilized for maximizing the *ex vivo* expansion of various types of hemopoietic cells. Such *ex vivo* expanded cells can be applied in several clinical situations. The following lists few.

Hemopoietic cell transplantation: Transplantation of hemopoietic cells has become the treatment of choice for a variety or inherited or malignant diseases. While early transplantation procedures utilized the entire bone marrow (BM) population, recently, more defined populations, enriched for stem cells (CD₃₄⁺ cells) have been used (1).

In addition to the marrow, such cells could be derived from other sources such as peripheral blood (PB) and neonatal umbilical cord blood (CB) (2). Compared to BM, transplantation with PB cells shortens the period of pancytopenia and reduces the risks of infection and bleeding (3-5).

An additional advantage of using PB for transplantation is its accessibility, The limiting factor for PB transplantation is the low number of circulating pluripotent stem/progenitor cells.

To obtain enough PB-derived stem cells for transplantation, these cells are "harvested" by repeated leukapheresis following their mobilization from the marrow into the circulation by treatment with chemotherapy and cytokines (3-4). Such treatment is obviously not suitable for normal donors.

The use of *ex vivo* expended stem cells for transplantation has the following advantages (2, 6-7).

It reduces the volume of blood required for reconstitution of an adult hemopoietic system and may obviate the need for mobilization and leukapheresis (3).

It enables storage of small number of PB or CB stem cells for potential future use.

In the case of autologous transplantation of patients with malignancies, contaminating tumor cells in autologous infusion often contribute to the recurrence of the disease (3). Selecting and expanding CD₃₄⁺ stem cells will reduce the load of tumor cells in the final transplant.

The cultures provide a significant depletion of T lymphocytes, which may be useful in the allogeneic transplant setting for reducing graft-versus-host disease.

Clinical studies have indicated that transplantation of *ex vivo* expanded cells derived from a small number of PB CD₃₄⁺ cells can restore hemopoiesis in patients treated with high doses of chemotherapy, although the results do not allow yet firm conclusion about the long term *in vivo* hemopoietic capabilities of these cultured cells (3-4).

For successful transplantation, shortening of the duration of the cytopenic phase, as well as long-term engraftment, is crucial. Inclusion of intermediate and late progenitor cells in the transplant could accelerate the production of donor-derived mature cells and shortens the cytopenic phase. It is important, therefore, that *ex vivo* expanded cells will include, in addition to stem cells, more differentiated progenitors in order to optimize short-term recovery and long term restoration of hemopoiesis. Expansion of intermediate and late progenitor cells, especially those committed to the neutrophilic and megakayocytic lineages, concomitant with expansion of stem cells, should serve this purpose (8).

Such cultures may be useful not only in restoring hematopoiesis in completely bone marrow ablated patients but also as supportive measure for shortening bone marrow recovery following conventional radio- or chemotherapies.

Prenatal diagnosis of genetic defects in scarce cells: Prenatal diagnosis involved the collection of embryonic cells from a pregnant woman and analysis thereof for genetic defects. A preferred, non-invasive, way of collecting embryonic cells involves separation of embryonic nucleated red blood cell precursors that infiltrated into the maternal blood circulation. However, being very scarce, such cells should undergo cell expansion prior to analysis. The present invention therefore offers means to expand embryonic cells for prenatal diagnosis.

Gene Therapy: For a successful long-term gene therapy a high frequency of transduced stem cells that have integrated the transgene into their genome is an obligatory requirement. In the BM tissue, while the majority of the cells are cycling progenitors and precursors, the stem cells constitute only a small fraction of the cell population and most of them are in a quiescent, non-cycling state.

Viral-based (e.g., retroviral) vectors require active cell division for integration of the transgene into the host genome. For these reasons gene transfer into fresh BM stem cells is very inefficient. The ability to expand a purified population of stem cells and to regulate their cell division *ex vivo* would permit increased probability of their transduction (9).

Adoptive Immunotherapy: *Ex vivo*-expanded, defined lymphoid subpopulations have been studied and used for adoptive immunotherapy of various malignancies, immunodeficiency, viral and genetic diseases (10-12).

The treatment enhances the required immune response or replaces deficient functions. This approach was pioneered clinically by Rosenberg et al. (13) using a large number of autologous *ex vivo* expanded non-specific killer T cells, and subsequently *ex vivo* expanded specific tumor infiltrating lymphocytes.

It was also shown that functionally active antigen-presenting cells can be grown from a starting population of CD₃₄⁺ PB cells in cytokine-supported cultures. These cells can present soluble protein antigens to autologous T cells *in vitro* and, thus, offer new prospects for the immunotherapy of minimal residual disease after high dose chemotherapy. *Ex vivo* expansion of antigen-presenting dendritic cells was also studied (14-16).

*Ex vivo* expansion of non-hemopoietic stem and progenitor cells: For example, *ex vivo* expansion of neural stem cells or oligodendrocyte progenitors.

Myelin disorders form an important group of human neurological diseases that are as yet incurable. Progress in animal models, particularly in transplanting cells of the oligodendrocyte lineage, has resulted in significant focal remyelination and physiological evidence of restoration of function (36). Future therapies could involve both transplantation and promotion of endogenous repair, and the two approaches could be combined with *ex vivo* manipulation of the donor tissue.

U.S. Pat. No. 5,486,359 teaches isolated human mesenchymal stem cells which can differentiate into more than one tissue type (e.g. bone, cartilage, muscle or marrow stroma) and a method for isolating, purifying, and culturally expanding human mesenchymal stem cells.

U.S. Pat. No. 5,736,396 teaches methods for *in vitro* or ex *vivo* lineage-directed induction of isolated, culture expanded human mesenchymal stem cells comprising the steps of contacting the mesenchymal stem cells with a bioactive factor effective to induce differentiation thereof into a lineage of choice. Further disclosed is a method which also includes introducing such culturally expanded lineage-induced mesenchymal stem cells into a host from which they have originated for purposes of mesenchymal tissue regeneration or repair.

U.S. Pat. No. 4,642,120 teaches compositions for repairing defects of cartilage and bones. These are provided in gel form either as such, or embedded in natural or artificial bones. The gel comprises certain types of cells. These may be committed embryonal chondocytes or any kind of mesenchyme originated cells which potentially can be converted to cartilage cells, generally by the influence of chondrogenic inducing factors, in combination with fibrinogen, antiprotease and thrombin.

U.S. Pat. No. 5,654,186 teaches that blood-borne mesenchymal cells proliferate in culture, and in vivo, as demonstrated in animal models, are capable of migrating into wound sites from the blood to form skin.

U.S. Pat. No. 5,716,411 teaches to a method of skin regeneration of a wound or burn in an animal or human. This method comprises the steps of initially covering the wound with a collagen glycosaminoglycan matrix, allowing infiltration of the grafted GC matrix by mesenchymal cells and blood vessels from healthy underlying tissue and applying a cultured epithelial autograft sheet grown from epidermal cells taken from the animal or human at a wound-free site on the animal's or human's body surface. The resulting graft has excellent take rates and has the appearance, growth, maturation and differentiation of normal skin.

U.S. Pat. No. 5,716,616 teaches methods of treating patients who are suffering from a disease, disorder or condition characterized by a bone cartilage or lung defects. The methods comprising the step of intravenous administration of stromal cells isolated from normal syngeneic individuals or intravenous administration of stromal cells isolated from the patient subsequent to correction of the genetic defect in the isolated cells. Methods of introducing genes into a recipient individual are also disclosed. The methods comprise the steps of obtaining a bone marrow sample from either the recipient individual or a matched syngeneic donor, isolating adherent cells from the sample, transfecting the adherent cells that were isolated from the recipient or a matched syngeneic donor with a gene and administering the transfected adherent cells to the recipient individual intravenously. Compositions that comprise isolated stromal cells that include exogenous genes operably linked to regulatory sequences are disclosed.

In each of the above examples, non-hemopoietic stem and progenitor cells are used as an external source of cells for replenishing missing or damaged cells of an organ. Such use requires cell expansion prior to differentiation in order to first obtain the required cell mass. It is in this step where the method of the present invention can become highly effective and useful while implementing any of the methods disclosed in the above U.S. patents.

Additional examples for both *ex vivo* and *in vivo* applications: skin regeneration, hepatic regeneration, muscle regeneration and bone growth in osteoporosis.

Mobilization of bone marrow stem cells into the peripheral blood (peripheralization): The discovery of the effect of transition metal chelators could also be applied *in vivo.* As mentioned above, PB-derived stem cells for transplantation are "harvested" by repeated leukapheresis following their mobilization from the marrow into the circulation by treatment with chemotherapy and cytokines (3-4).

The use of chemotherapy is, of course, not suitable for normal donors. Administration of transition metal chelators, such as TEPA, into the donor could increase the marrow stem cell pool, which is then mobilized into the periphery by endogenous or injected G-CSF.

Leukemia: Unlike normal hematopoiesis, in leukemia, the processes of proliferation and differentiation are uncoupled; the malignant cells are unable to differentiate and consequently maintain continuous proliferation ability.

Understanding of the molecular events driving the uncoupling of the proliferation and differentiation processes of normal progenitors following transition metals depletion, in particular Copper, may shed light on the cellular processes involved in the development of leukemia.

Stimulation of fetal hemoglobin production: Increased fetal hemoglobin has been shown to ameliorate the clinical symptoms in patients with β-hemoglobinopathies such as sickle cell anemia and β-thalassemia (38).

Fetal hemoglobin, which normally comprises about 1 % of the total hemoglobin, becomes elevated in accelerated erythropoiesis (e.g., following acute hemolysis or hemorrhage or administration of erythropoietin) (35).

It has been suggested that this phenomenon is associated with acceleration of the maturation/differentiation process of the erythroid precursors (37).

Administration of transition metal chelators such as TEPA to patients with β-hemoglobinopathies might first increase and synchronize their early erythroid progenitor pool (by blocking differentiation).

Following cessation of administration of the drug and its removal from the body, this early population then might undergo accelerated maturation which may result in elevated production of fetal hemoglobin.

Thus, according to the present invention there is provided a method of expanding a population of cells *ex-vivo*, while at the same time inhibiting differentiation of the cells. The method includes the step of providing the cells with conditions for cell proliferation and, at the same time, reducing a capacity of the cells in utilizing transition metals, such as Copper.

Reducing the capacity of the cells in utilizing transition metals may be effected, for example, either by depletion thereof (e.g., via suitable chelators) or by interference in their metabolism (e.g., via addition of Zinc ions).

As used herein the term "inhibiting" refers to slowing, decreasing, delaying, preventing or abolishing.

As used herein the term "differentiation" refers to change from relatively generalized to specialized kinds during development. Cell differentiation of various cell lineages is a well documented process and requires no further description herein.

Administration of the transition metal chelator and/or Zinc ions may be by a pharmaceutical composition including same, which may further include thickeners, carriers, buffers, diluents, surface active agents, preservatives, and the like, all as well known in the art.

The pharmaceutical composition may be administered in either one or more of ways depending on whether local or systemic treatment is of choice, and on the area to be treated. Administration may be done topically (including ophtalmically, vaginally, rectally, intranasally), orally, by inhalation, or parenterally, for example by intravenous drip or intraperitoneal, subcutaneous, intramuscular or intravenous injection.

Formulations for topical administration may include but are not limited to lotions, ointments, gels, creams, suppositories, drops, liquids, sprays and powders. Conventional pharmaceutical carriers, aqueous, powder or oily bases, thickeners and the like may be necessary or desirable.

Compositions for oral administration include powders or granules, suspensions or solutions in water or non-aqueous media, sachets, capsules or tablets. Thickeners, diluents, flavorings, dispersing aids, emulsifiers or binders may be desirable.

Formulations for parenteral administration may include but are not limited to sterile solutions which may also contain buffers, diluents and other suitable additives.

Dosing is dependent on severity and responsiveness of the condition to be treated, but will normally be one or more doses per day, with course of treatment lasting from several days to several months or until a cure is effected or a diminution of disease state is achieved. Persons ordinarily skilled in the art can easily determine optimum dosages, dosing methodologies and repetition rates. Slow release administration regime may be advantageous in some applications.

According to the present invention the cells to be expanded are present *ex vivo.*

As used herein the term "*ex vivo*" refers to cells removed from a living organism and are propagated outside the organism (e.g., in a test tube). As used herein, the term "*ex vivo*", however, does not refer to cells known to propagate only *in vitro,* such as various cell lines (e.g., HL-60, HeLa, etc.).

Providing the *ex vivo* grown cells with the conditions for cell proliferation include providing the cells with nutrients and preferably with one or more cytokines. Again, reducing the capacity of the cells in utilizing transition metals, such as Copper is effected by a suitable transition metal chelator and/or Zinc ions.

Final concentrations of the chelator and/or Zinc ions may be, depending on the specific application, in the micromolar or milimolar ranges. For example, within about 0.1 µM to about 100 mM, preferably within about 4 µM to about 50 mM, more preferably within about 5 µM to about 40 mM.

The chelator may be a polyamine chelating agent, such as, but not limited to ethylendiamine, triethylenetetramine, tetraethylenepentamine, pentaethylenehexamine, captopril or penicilamine, preferably tetraethylpentamine.

The above listed chelators are known in their high affinity towards Copper ions. However, these chelators have a substantial affinity also towards other transition metals (39).

According to another preferred embodiment of the invention the cytokines are early acting cytokines, such as, but not limited to, stem cell factor, FLT3 ligand, interleukin-6, thrombopoietin and interleukin-3, and/or late acting cytokines, such as, but not limited to, granulocyte colony stimulating factor, granulocyte/macrophage colony stimulating factor and erythropoietin.

The cells are hematopoietic cells.

Depending on the application, hematopoietic cells may be obtained for *ex vivo* expansion according to the method of the present invention from bone marrow, peripheral blood, or neonatal umbilical cord blood.

Preferably, the hematopoietic cells are enriched for hemopoietic CD₃₄+ cells (i.e., stem cells). Enriching the fraction of stem cells may be effected by cell sorting, as well known in the art.

The cells expanded according to the present invention may be either non-differentiated stem cells or committed progenitor cells. Stem cells are known for many cell lineages. These cells are characterized by being the most undifferentiated cells of the lineage. Progenitor cells, on the other hand, are more differentiated, as they are already committed to a differentiation path within the cell lineage.
Further according to the present invention there is provided a method of transducing (transfecting, transforming) expanded, undifferentiated therapeutic hematopoietic stem and progenitor cells or further characterized in the claims.

Transduction methods are well known in the art and require no further description herein. Examples of transduction protocols are found in many laboratory manuals including Sambrook, J., Fritsch, E.F., Maniatis, T. (1989) Molecular Cloning. A Laboratory Manual. Cold Spring
Further according to the present invention there is provided a therapeutical *ex vivo* cultured hematopoietic stem and progenitor cell population as further characterized in the claims.

Reference is now made to the following examples, which together with the above descriptions, illustrate the invention in a non limiting fashion.

### EXAMPLE 1

### Experimental Procedures

***CD₃₄ cells selection:*** Peripheral blood "buffy coat" cells derived from a whole blood unit, peripheral blood cells obtained following leukapheresis, or cord blood cells where layered on Ficoll-Hypaque (density 1.077 g/ml) and centrifuged at 1,000 x g for 20 min. at room temperature. The interphase layer of mononuclear cells were collected, washed three times with Ca/Mg free phosphate buffered saline containing 1 % bovine serum albumin (BSA). The cells were incubated for 30 min. at 4 °C with murine monoclonal anti CD₃₄ antibody (0.5 µ g/10⁶ mononuclear cells) and thereafter isolated using the miniMACS apparatus (Miltenyi-Biotec, Bergisch, Gladbach, Germany) according to the manufacturer's protocol.

***Culture procedures:*** For the expansion of progenitor cells, CD₃₄⁺ enriched fractions or unseparated mononuclear cells were seeded at about 1-3x10⁴ cells/ml in either alpha minimal essential medium containing 10 % preselected fetal calf serum (FCS) (both from GIBCO, Grand Island, NY), or serum-free medium (Progenitor-34 medium, Life Technologies, Grand Island, NY). The media were supplemented with a mixture of growth factors and transition metal chelators. The cultures were incubated at 37 °C in an atmosphere of 5 % CO₂ in air with extra humidity. Half of the medium was changed weekly with fresh medium containing all the supplements.

***Cloning potential evaluations:*** The cloning potential of cells developed in the liquid culture was assayed, at different intervals, in semi-solid medium. The cells were washed and seeded in 35 mm dishes in methylcellulose containing alpha medium supplemented with recombinant growth factors (SCF, G-CSF, GM-CSF and EPO). Following 2 weeks incubation, the cultures were scored with an inverted microscope. Colonies were classified as blast, mixed, erythroid, myeloid, and megakaryocytic, according to their cellular composition.

***Morpholigical assessment:*** In order to characterize the resulting culture populations, aliquots of cells were deposited on a glass slide (cytocentrifuge, Shandon, Runcorn, UK), fixed and stained in May-Grunwald Giemsa. Other aliquots were stained by benzidine for intracellular hemoglobin.

***Immunofluorescence staining:*** At different intervals, cells from the liquid cultures were assayed for CD₃₄ antigen. Aliquots were harvested, washed and incubated on ice with FITC-labeled anti CD₄₅ monoclonal antibody and either PE-labeled anti CD₃₄ (HPCA-2) monoclonal antibody or PE-labeled control mouse Ig. After incubation, red cells were lysed with lysing solution, while the remaining cells were washed and analyzed by flow cytometer.

***Flow cytometry:*** Cells were analyzed and sorted using FACStar^{plus} flow cytometer (Becton-Dickinson, Immunofluorometry systems, Mountain View, CA). Cells were passed at a rate of 1,000 cells/second through a 70 mm nozzle, using saline as the sheath fluid. A 488 nm argon laser beam at 250 mW served as the light source for excitation. Green (FITC-derived) fluorescence was measured using a 530±30 nm band-pass filter and red (PE-derived) fluorescence - using a 575±26 nm band filter. The PMTs was set at the appropriate voltage. Logarithmic amplification was applied for measurements of fluorescence and linear amplification - for forward light scatter. At least 10⁴ cells were analyzed.

### EXAMPLE 2

### Experimental Results

In an effort to develop culture conditions which stimulate proliferation and inhibit differentiation of hemopoietic progenitor cells, CD₃₄⁺ cells were cultured with the following supplements:
Transition metal chelators such as - tetraethylpentamine (TEPA), captopril (CAP) penicilamine (PEN) or other chelators or ions such as Zinc which interfere with transition metal metabolism;
Early-acting cytokines - stem cell factor (SCF), FLT3 ligand (FL), interleukin-6 (IL-6), thrombopoietin (TPO) and interleukin-3 (IL-3);
Late-acting cytokines - granulocyte colony stimulating factor (G-CSF), granulocyte/macrophage colony stimulating factor (GM-CSF) and erythropoietin (EPO).

***TEPA effects on proliferation and clonability of short term CD₃₄+ cultures:*** Addition of TEPA to CD₃₄⁺ cells cultured with low doses of early-acting cytokines resulted in a significant increase in total cell number, in the number of CD₃₄⁺ cells (measured by flow cytometry utilizing fluorescence labeled specific antibodies, Figure 2) and in cell clonability (measured by plating culture aliquots in semi-solid medium and scoring colonies that develop two weeks later, Figure 1), compared to cultures supplemented only with cytokines. The colonies which developed in semi-solid medium in the presence of TEPA were of myeloid, erythroid and mixed phenotype.

The effects of TEPA were further assessed in cultures supplemented with either high doses of early cytokines (Table 1) or with a combination of early- and late-acting cytokines (Figure 3). The results indicated that TEPA significantly increased the clonability and the percentage of CD₃₄⁺ cells in these cultures. As for total cell number it was increased by TEPA in cultures supplemented with early cytokines (Table 1; Figure 2), whereas in cultures supplemented with both early and late cytokines, TEPA caused a marginal inhibition (Figure 3).

**TABLE 1**

| ***The short-term effect of TEPA on CD₃₄ cells*** | | | | | |
|---|---|---|---|---|---|
| TEPA | Il-3 | Cells/ml (x10⁴) | CD₃₄ cells (%) | Colonies (Per 1x10³ initiating cells) | CFU expansion (fold) |
| - | - | 1 | 1 | 16 | 0.3 |
| + | - | 2 | 11.5 | 140 | 2.8 |
| - | + | 5 | 5 | 165 | 3.3 |
| + | + | 11 | 20 | 850 | 17 |

Cord blood-derived CD₃₄ cells were plated in liquid culture in the presence of: FLT 3 ligand - 50ng/ml, SCF - 50ng/ml, IL-6 - 50ng/ml, with or without IL-3 - 20ng/ml, with or without TEPA - 10 µM. On day 7, the percentage of CD₃₄ cells and the total cell number were determined Aliquots equivalent to 1x10³ initiating cells were assayed on days 0 and 7 for colony forming cells (CFU) by cloning in semi-solid medium. CFU expansion represents the ratio of CFU present on day 7 to CFU present on day 0.

***TEPA on proliferation and clonability of long-term CD₃₄*⁺ cultures:** Long-term cultures were maintained for 3-5 weeks by weekly demi-depopulation (one half of the culture volume was removed and replaced by fresh medium and cytokines). Addition of TEPA resulted in a higher clonability in long-term cultures supplemented with either early cytokines (Figure 4) or both early and late cytokines (Figure 3), as compared to cultures supplemented only with cytokines.

After three weeks in culture, there was a sharp decrease in clonability in cultures supplemented only with cytokines, whereas cultures treated with TEPA in combination with cytokines maintained high clonability, which was even higher than that of short-term cultures.

***The effect of TEPA on the maturation of hematopoietic cells:*** The effect of TEPA on the maturation of hematopoietic cells was tested on several models:

Mouse erythroleukemic cells (MEL): MEL cells are erythroblast like cells. Following treatment with several chemicals (differentiation inducers) the cells undergo erythroid differentiation and accumulate hemoglobin. MEL cells were cultured in the presence of the differentiation inducer hexamethylene bisacetamide (HMBA) and the chelators TEPA or Captopril. At day 3 of the culture, the total number of cells and the percentage of hemoglobin-containing cells were determined (Table 2). The results indicated that both TEPA and captopril inhibited the HMBA-induced differentiation of MEL cells.

Human erythroid cell cultures: Normal human erythroid cells were grown according to the two-phase liquid culture procedure, essentially as described in references 23-26. In the first phase, peripheral blood mononuclear cells were incubated in the presence of early growth factors for 5-7 days. In the second phase, these factors were replaced by the erythroid specific proliferation/differentiation factor, erythropoietin.

The cultures were supplemented with TEPA at the initiation of the second phase. The total cell number and the percentage of hemoglobin-containing cells were determined after 14 days. The results (Figure 5) showed that in the presence of TEPA there was a sharp decrease in hemoglobin-containing cells, while the total number of cells decreased only slightly.

These results suggest that TEPA inhibits erythroid differentiation, but does not significantly affect the proliferation ability of the progenitor cells.

**TABLE 2**

| **The effect of TEPA and captopril on growth and differentiation of erythroleukemia cells** | | |
|---|---|---|
| | Cells/ml (x10⁴) | Benzidine Positive Cells (%) |
| Control | 31 | <1 |
| HMBA | 32 | 46 |
| HMBA + TEPA 5 µM | 35 | 24 |
| HMBA + TEPA 10 µM | 35 | 16 |
| HMBA + TEPA 20 µM | 47 | 16 |
| HMBA + Captopril 20 µM | 34 | 29 |
| HMBA + Captopril 40 µM | 34 | 12 |

Murine erythroleukemia cells (MEL), were cultured in liquid medium supplemented with the differentiation inducer - hexamethylene-bisacetamide (HMBA, 4 mM), with or without different concentrations of TEPA or captopril. On day 3, total cell number and hemoglobin containing (benzidine positive) cells were determined.

CD₃₄+ initiated cultures: Long term liquid cultures initiated with CD₃₄+ cells were maintain with different cocktails of cytokines. Half of the cultures were continuously supplemented with TEPA. In order to test the status of cell differentiation, cytospin preparation were stained with May-Grunwald Giemsa (Figures 6a-d). The results showed that cultures which were maintained for 4-5 weeks without TEPA contained only fully differentiated cells, while with TEPA the cultures contained, in addition to fully differentiated cells, a subset of 10 % - 40 % of undifferentiated blast-like cells.

These results strongly suggest that TEPA induces a delay in CD₃₄⁺ cell differentiation which results in prolonged proliferation and accumulation of early progenitor cells in long-term *ex vivo* cultures.

*TEPA's mechanism of activity:* In order to determine whether TEPA affects CD₃₄⁺ cells via depletion of transition metals, such as Copper, two approaches were taken.

The first was to assess the effect of different transition metal chelators: tetra-ethylpentamine (TEPA), captopril (CAP) or penicilamine (PEN). The results demonstrated that all these compounds share the same effects on CD₃₄⁺ cells as TEPA (Figure 7).

The second approach was to supplement TEPA-treated cultures with Copper. The results indicated that TEPA activities were reversed by Copper (Figure 8), while supplementation with other ions, such as iron and selenium, did not (Figure 9), at least in the short to medium term cultures employed herein.

Zinc, which is known to interfere with transition metal metabolism, e.g., with Copper metabolism, expand the clonability of the cultures by itself. This effect was even more pronounced in the presence of both Zinc and TEPA (Figure 10).

In the above examples it is demonstrated that by supplementing CD₃₄ cell cultures with early-acting cytokines and the polyamine agent - tetraethylenepentamine (TEPA), for example, it is possible to maintain long term cultures (LTC) without the support of stroma. Three phenomena were evident in these cultures: (i) continuos cell proliferation; (2) expansion of clonogenic cells (CFUc); and (iii) maintenance of cells at their undifferentiated status.

In contrast, control, TEPA-untreated cultures ceased to proliferate and to generate CFUc and their cells underwent differentiation much earlier.

Thus, TEPA and other transition metal chelators sustains long-term cultures by inhibiting/delaying cellular differentiation through chelation of transition metals, Copper in particular.

The following Example No. 3 further substantiate the results described hereinabove; teaches optimal culture conditions for long-term cultures, teaches additional chelating agents that affect hemopoietic cell differentiation and sheds more light on the mechanism of activity of TEPA and other chelators on their target cells.

### EXAMPLE 3

CD₃₄⁺ cells derived from human neonatal cord blood were purified by immunomagnetic method and then cultured in liquid medium supplemented with cytokines either with or without transition metal chelators. At weekly intervals, the cultures were demi-depopulated by removing half of the culture content (supernatant and cells) and replacing it with fresh medium, cytokines and the chelators. At the indicated weeks the cellular content of the cultures were quantitated for total cells (by a manual microscopic/hemocytometric method), for CD₃₄⁺ cells (by immuno-flow cytometry) and for clonogenic cells (by cloning the cells in cytokine-supplemented semi-solid medium). The cultures were initiated with 1x10⁴ cells, 50-80 % of which were CD₃₄⁺ and 25-50 % of which were CFUc. The results presented in Figures 11 to 24 were calculated per 1x10⁴ initiating cells (the numbers were multiplied by the dilution factors).

Figure 11 shows the effect of TEPA on long-term CD₃₄ cultures. Cultures initiated with CD₃₄ cells in liquid medium supplemented with early-acting cytokines (in the absence of stromal cells) could be maintained by TEPA for a long time (>6 weeks). In such cultures, TEPA supported, in combination with the cytokines, maintenance and expansion of clonogenic cells (CFUc): The cultures were started with 2.5x10³ CFUc. Upon termination after 6 weeks, TEPA-treated cultures contained 300x10³ CFUc, (i.e., a 120-fold expansion) while control cultures contained no CFUc.

Figures 12-14 show the effect of TEPA on cell proliferation, CFUc and CFUc frequency in the presence of different combination of early cytokines. The combination of the early-acting cytokines TPO, SCF, FLT 3 ligand IL-6 and TEPA was found to be the optimal combination for the maintenance and long term expansion of cells with clonogenic potential.

Figure 15 shows the effect of G-CSF and GM-CSF on CFUc frequency of control and TEPA-supplemented CD₃₄ cultures. Supplementing the cultures with the late-acting cytokines G-CSF and GM-CSF, which stimulate cell differentiation, resulted in rapid loss of clonogenic cells. This differentiation stimulatory effect is blocked by TEPA.

Figures 16-17 show the effect of partial or complete medium + TEPA change on long-term cell proliferation (Figure 16) and CFUc production (Figure 17). The results obtained indicate that for maintaining maximal expansion, TEPA should be completely replaced, at least, at weekly intervals.

Figure 19 shows the effect of delayed addition of TEPA on CFUc frequency. It is evident that early exposure of CD₃₄ cells to TEPA was crucial for long-term maintenance and expansion of CFUc, suggesting that TEPA affects differentiation of progenitors at various stages of differentiation.

Figure 20 shows the effect of short-term preincubation with a single cytokine on long-term CFUc production. The results indicate that LTC-CFC are more preserved in TEPA-treated cultures when supplemented for the first 24 hours with a single cytokine rather than the full complement of cytokines, suggesting that under the former conditions cells are blocked more efficiently.

Figures 21 a-b show the effect of polyamine chelating agents on CD₃₄ cell cultures. Polyamine chelating agents sustained cell proliferation and expanded CFUc during long term cultures. Among the compounds tested, the long-chain polyamines, TEPA and PEHA, were found to be more effective,than the short-chain polyamines.

Figures 22a-b show the effect of transition metal chelating agents on CD₃₄ cell cultures. Penicilamine (PEN) and captopril (CAP), which are known transition metal chelators, sustained cell proliferation and expansion of clonogenic cells during long-term cultures.

Figure 23a-b show the effect of Zinc on CD₃₄ cell cultures. Zinc, which is known to interfere with transition metal metabolism, Copper in particular, mimicked the effect of the chelating agents in long term cultures, but to a smaller extent than the chelators themselves.

Thus, *ex vivo* expansion of hematopoietic progenitor cells is limited by the progression of these cells into non-dividing differentiated cells. This differentiation process can be delayed by cultivating the progenitor cells on stroma cell layer. Since the stroma supports continuous cell proliferation and long-term generation of CFUc, it is believed that the stroma inflict an anti differentiation effect on the progenitor cells.

We have developed a novel system which sustains continuous cell proliferation and long-term generation of CFUc in stroma-free cultures (Figure 11). The system combines the use of early-acting cytokines, such as stem cell factor (SCF), FLT3 ligand, interleukin-6 (IL-6), thrombopoietin (TPO) with or without interleukin-3, and transition metal chelating agents (Figures 12-14). The early cytokines support the survival and proliferation of the progenitors with reduced stimulus for differentiation compared to late-acting cytokines, such as G-CSF and GM-CSF (Figure 15). The chelators inhibit differentiation through chelation of transition metals, Copper in particular. Complete medium change at weekly intervals, as compared to partial change, improved LTC-CFC maintenance, suggesting that the TEPA-transition metal complex, e.g., TEPA-Copper complex, may not be stable (Figures 16-17).

Several lines of evidence suggest that TEPA inhibits differentiation of early progenitors (Figure 18). For example, when TEPA addition was delayed until day 6 of the culture its effects were reduced as compared to cultures supplemented with TEPA from day 1 (Figure 19).

While optimal results were obtained when TEPA was added on day 1, it was advantageous to add the full complement of cytokines on day 2. Thus, TEPA-treated cultures which were supplemented for one day with only one cytokine, e.g., FLT3 ligand followed by addition of the other cytokines (SCF, TPO and IL-3) were maintained longer than cultures where all the cytokines were added at day 1 (Figure 20). We hypothesize that since cell differentiation is driven by the cytokines and is dependent on Copper and other transition metals, inhibition of differentiation requires depletion thereof prior to exposure to the full complement of cytokines. A single cytokine does not support rapid activation (proliferation and differentiation) but maintains cell viability, thus allowing TEPA to efficiently chelate transition metals in quiescent undifferentiated CD₃₄ cells prior to activation.

Following screening, various chelating agents have been found to support continuous cell proliferation and long-term generation of CFUc and to delay cell differentiation. Among them are the polyamines such as, but not limited to, TEPA, EDA, PEHA and TETA (Figures 21a-b) or chelators such as, but not limited to, penicilamine (PEN) and captopril (CAP) (Figures 22a-b). Zinc which interfere with transition metals (Copper in particular) metabolism also supported LTC-CFC (Figures 23a-b).

### EXAMPLE 4

Further described herein is a method of preservation of stem cells, such as, but not limited to, cord blood derived stem cells, peripheral blood derived stem cells and bone marrow-derived stem cells. The method is effected by handling the stem cell while being harvested, isolated and/or stored, in a presence of a transition metal chelator, e.g., TEPA.

Cord blood-derived cells were collected and stored (unseparated) for 24 hours, at 4 °C, either in the presence or absence of 10 µM TEPA. CD₃₄⁺ cells were then separated using either 10 µM TEPA-PBS buffer or TEPA free PBS buffer, respectively. Then, cells were grown in long-term cultures in the presence of 10 µM TEPA.

The results indicated that cultures which were initiated with cells that were handled in the presence of TEPA expanded for 8 weeks, whereas cultures initiated from cells stored without TEPA stopped expanding after 5 weeks only.

It is well known that it takes usually at least several hours between cell collection and either freezing or transplantation.

These results indicate that addition of a transition meta chelator, such as TEPA, to the collection bags and the separation and washing buffers increase the yield of stem cells and improve their potential for long-term growth, thus facilitate the short-term take and the long-term repopulation following transplantation of either "fresh", cryopreserved or ex-vivo expanded hemopoietic cells.

Further described herein are stem cell collection bags and separation and washing buffers supplemented with an effective amount or concentration of transition metal chelator, which inhibits differentiation.

### LIST OF REFERENCES CITED

1. Van Epps DE, et al. Harvesting, characterization, and culture of CD34+ cells from human bone marrow, peripheral blood, and cord blood. Blood Cells 20:411, 1994.
2. Emerson SG. Ex vivo expansion of hematopoietic precursors, progenitors, and stem cells: The next generation of cellular therapeuties. Blood 87:3082, 1996.
3. Brugger W, et al. Reconstitution of hematopoiesis after high-dose chematotherapy by autologus progenitor cells generated in vivo. N Engl J Med 333:283, 1995.
4. Williams SF, et al. Selection and expansion of peripheral blood CD34+ cells in autologous stem cell transplantation for breast cancer. Blood 87:1687, 1996.
5. Zimmerman RM, et al. Large-scale selection of CD34+ peripheral blood progenitors and expansion of neutrophil precursors for clinical applications. J Heamatotherapy 5:247, 1996.
6. Koller MR, Emerson SG, Palsson BO. Large-scale expansion of human stem and progenitor cells from bone marrow mononuclear cells in continuous perfusion cultures. Blood 82:378, 1993.
7. Lebkowski JS, et al. Rapid isolation and serum-free expansion of human CD34+ cells. Blood Cells 20:404, 1994.
8. Sandstrom CE, et al. Effects of CD34+ cell selection and perfusion on ex vivo expansion of peripheral blood mononuclear cells. Blood 86:958, 1995.
9. Eiprs PG, et al. Retroviral infection of primitive hematopoietic cells in continuous perfusion culture. Blood 86:3754, 1995.
10. Freedman AR, et al. Generation of T lymphocytes from bone marrow CD34+ cells in vitro. Nature Medicine 2:46, 1996.
11. Heslop HE, et al. Long term restoration of immunity against Epstein-Barr virus infection by adoptive transfer of gene-modified virus-specific T lymphocytes. Nature Medicine 2:551, 1996.
12. Protti MP, et al. Particulate naturally processed peptides prime a cytotoxic response against human melanoma in vitro. Cancer Res 56:1210, 1996.
13. Rosenberg SA, et al. Prospective randomized trial of high-dose interleukin-2 alone or in conjunction with lymphokine-activated killer cells for the treatment of patients with advanced cancer. J Natl Cancer Inst 85: 622, 1993.
14. Bernhard H, et al. Generation of immunostimulatory dendritic cells from human CD34+ hematopoietic progenitor cells of the bone marrow and peripheral blood. Cancer Res 1099, 1995.
15. Fisch P, et al. Generation of antigen-presenting cells for soluble protein antigens ex vivo from peripheral blood CD34+ hematopoietic progenitor cells in cancer patients. Eur J Immunol 26:595, 1996.
16. Siena S, et al. Massive ex vivo generation of functional dendritic cells from mobilized CD34+ blood progenitors for anticancer therapy. Expt Hematol 23:1463, 1996.
17. Petzer AL, Zandstra PW, Piret JM, Eaves CJ. Differential cytokine effects on primitive (CD34+CD38-) human hematopoietic cells: novel responses to F1T3-ligand and thrombopoietin. J Exp Med 183:2551, 1996.
18. Schwartz RM, et al. In vitro myelopoiesis stimulated by rapid medium exchange and supplementation with hematopoietic growth factors. Blood 78:3155, 1991.
19. Verfaillie CM. Can human hematopoietic stem cells be cultured in vivo? Stem Cells 12:466, 1994.
20. Haylock DN, et al. Ex vivo expansion and maturation of peripheral blood CD34+ cells into the myeloid lineage. Blood 80:1405, 1992.
21. Brugger W, et al. Ex vivo expansion of enriched peripheral blood CD34+ progenitor cells by stem cell factor, interleukin-1 beta (IL-1 beta), IL-6, IL-3, interferon-gamma, and erythropoietin. Blood 81:2579, 1993.
22. Sato N, et al. In vitro expansion of human peripheral blood CD34+ cells. Blood 82:3600, 1993.
23. Fibach E, Manor D, Oppenheim A, Rachmilewitz EA. Proliferation and maturation of human erythroid progenitors in liquid medium. Blood 73:100, 1989.
24. Fibach E, Manor D, Treves A, Rachmilewitz EA. Growth of human normal erythroid progenitors in liquid culture: A comparison with colony growth in semisolid culture. Internatl J Cell Clon 9:57, 1991.
25. Fibach E, Rachmilewitz EA. The two-step liquid culture - novel procedure for studying maturation of human normal and pathologic erythroid precursors. Stem Cells 11:36, 1993.
26. Dalyot N, Fibach E, Rachmilewitz E, Oppenheim A. Adult and neonatal patterns of human globin gene expression are recapitulated in liquid cultures. Exper Hematol 20:1141, 1992.
27. Banno S, et al. Anemia and neutropenia in elderly patients caused by copper deficiency for long-term eternal nutrition. Rinsho-ketsueki 35:1276, 1994.
28. Wasa M, et al. Copper deficiency with pancytopenia during total parenteral nutrition. JPEN J Parenter Enteral Nutr 18:190, 1994.
29. Zidar BL, Shadduck RK, Zeigler Z, Winkelstein A. Observation on the anemia and neutropenia of human copper deficiency. Am J Hematol 3:177, 1977.
30. Hirase N, et al. Anemia and neutropenia in a case of copper deficiency: Role of copper in normal hematopoiesis. Acta Haematol 87:195, 1992.
31. Percival SS, Layden-Patrice M. HL-60 cells can be made copper deficient by incubating with tetraethylenepentamine. J Nutr 122:2424, 1992.
32. Percival SS. Neutropenia caused by copper deficiency: possible mechanisms of action. Nutr Rev 53:59, 1995.
33. Bae B, Percival SS. Retinoic acid-induced HL-60 cell differentiation is augmented by copper supplementation. J Nutr 123:997, 1993.
34. Bae B, Percival SS. Copper uptake and intracellular distribution during retinoic acid-induced differentiation of HL-60 cells. J Nutr Biochem 5:457, 1994.
35. Alter BP. Fetal erythropoiesis in stress hemopoiesis. Experimental Hematology 7:200, 1979.
36. Repair of myelin disease: Strategies and progress in animal models. Molecular Medicine Today. Dec. 1997. pp. 554-561.
37. Blau CA et al. Fetal hemoglobin in acute and chronic stage of erythroid expansion. Blood 81:227, 1993.
38. Schechtez AN et al. Sickle cell anemia. In: Molecular basis of blood diseases. Stamatoyannaopoulos G, Nienhuis AW, Leder P and Majerus PW Eds. pp. 179-218, Sounders Philadelphia.
39. Ross JW and Frant MS. Chelometric indicators, titration with the solid state cupric ion selective electrode. Analytical Chemistry 41:1900, 1969.
40. Fosmire GJ. Zinc toxicity. Am J Clin Nutr 51 (2): 225-227, 1990.
41. Simon SR, et al. Copper deficiency and siberoblastic anemia associated with zinc ingestion. Am J Hematol 28(3): 181-183,1988.
42. Hoffman HN 2d, et al. Zinc-induced copper deficiency. Gastroenterology 94(2): 508-512, 1988.
43. Reeves PG, et al. High zinc concentrations in culture media affect copper uptake and transport in differentiated human colon adenocarcinoma cells. J Nutr 126(6): 1701-1712, 1996.
44. Tashiro Itoh T, et al. Metallothionein expression and concentrations of copper and zinc are associated with tumor differentiation in hepatocellular carcinoma. Liver 17(6): 300-306, 1997.
45. Cable EE, Isom HC. Exposure of primary rat hepatocytes in long-term DMSO culture to selected transition metals induces hepatocyte proliferation and formation of duct-like structures. Hepatology 26(6): 1444-1457, 1997.
46. Kizaki M, et al. Regulation of manganese superoxide dismutase and other antioxidant genes in normal and leukemic hematopoietic cells and their relationship to cytotoxicity by tumor necrosis factor. Blood 82(4): 1142-1150, 1993.
47. Brugnera E, et al. Cloning, chromosomal mapping and charatcerization of the human metal-regulatory transcription factor MTF-1. Nucleic Acids Res 22(15): 3167-3173, 1994.
48. Heuchel R, et al. The transcription factor MTF-1 is essential for basal and heavy metal-induced metallothionein gene expression. Embo J 13(12): 2870-2875, 1994.
49. Palmiter RD. Regulation of metallothionein genes by heavy metals appears to be mediated by a zinc-sensitive inhibitor that interacts with a constitutively active transcription factor, MTF-1. Proc Natl Acad Sci USA 91(4): 1219-1223, 1994.
50. Hatayama T, et al. Regulation of hsp70 synthesis induced by cupric sulfate and zinc sulfate in thermotolerant HeLa cells. J Biochem Tokyo 114(4): 592-597, 1993.
51. Filvaroff E, et al. Functional evidence for an extracellular calcium receptor mechanism triggering tyrosine kinase activation associated with mouse keratinocyte differentiation. J Biol Chem 269(34):21735-21740, 1994.
52. Okazaki T, et al. Characteristics and partial purification of a novel cytosolic, magnesium-independent, neutral sphingomyelinase activated in the early signal transduction of a 1 alpha, 25-dihydroxyvitamin D3-induced HL-60 cell differentiation. J Biol Chem 269(6): 4070-4077, 1994.]
53. Kim H, Lipscomb WN. Differentiation and identification of the two catalytic metal binding sites in bovine lens leucine aminopeptidase by x-ray crystallography. Proc Natl Acad Sci USA 90(11): 5006-5010, 1993.

## Claims

1. A method of expanding hematopoietic stem and progenitor cells *ex-vivo,* while at the same time inhibiting differentiation of said hematopoietic stem and progenitor cells, the method comprising providing said hematopoietic stem and progenitor cells *ex-vivo* with conditions for cell proliferation and with a transition metal chelator selected from the group consisting of tetraethylenepentamine (TEPA), ethylenediamine (EDA), pentaethylenehexamine (PEHA), triethylenetetramine (TETA), penicilamine and captopril, wherein said conditions for cell proliferation comprise nutrients and at least an early-acting cytokine or early-acting cytokines, wherein said hematopoietic stem and progenitor cells are therapeutic hematopoietic stem and progenitor cells, and wherein said chelator and said proliferation conditions result in
(i) prolonged active proliferation;
(ii) long-term expansion of clonogenic cells (CFUc); and
(iii) maintenance of undifferentiated cells in their undifferentiated state;
with the proviso that said hematopoietic cells are not human embryonic stem cells obtained from a human embryo.

2. The method of claim 1, wherein said transition metal chelator is TEPA.

3. The method of claim 1, wherein said transition metal chelator concentration is 0.1 µM to 100 mM.

4. The method of claim 3, wherein said transition metal chelator concentration is 4 µM to 50 mM.

5. The method of claim 4, wherein said transition metal chelator concentration is 5 µM to 40 mM.

6. The method of claim 1, wherein said conditions for cell proliferation further comprise providing a late acting cytokine or late acting cytokines.

7. The method of claim 6, wherein said late acting cytokine is selected from the group consisting of granulocyte colony stimulating factor, granulocyte/macrophage colony stimulating factor and erythropoietin.

8. The method of claim 6, wherein said late acting cytokine is granulocyte colony stimulating factor.

9. The method of claim 1, wherein said hematopoietic cells are derived from a source selected from the group consisting of bone marrow cells, peripheral blood cells and neonatal umbilical cord blood cells.

10. The method of claim 1, wherein said hematopoietic stem and progenitor cells are derived from neonatal umbilical cord blood cells.

11. The method of claim 1, further comprising the step of selecting a population of hematopoietic cells enriched for hematopoietic stem cells.

12. The method of claim 11, wherein said cells are enriched for CD34+.

13. A method of transducing expanded, undifferentiated therapeutic hematopoietic stem and progenitor cells with an exogene, the method comprising:
(a) expanding and inhibiting differentiation of said hematopoietic stem and progenitor cells by:
(i) providing said hematopoietic stem and progenitor cells with conditions for cell proliferation, wherein said conditions for cell proliferation comprise nutrients and at least an early acting cytokine or early-acting cytokines and wherein said hematopoietic stem and progenitor cells are therapeutic hematopoietic stem and progenitor cells;
(ii) contacting said hematopoietic stem and progenitor cells with a transition metal chelator selected from the group consisting of TEPA, EDA, PEHA, TETA, penicilamine and captopril, wherein said chelator and said proliferation conditions result in prolonged active proliferation; long-term expansion of clonogenic cells (CFUc); and maintenance of undifferentiated cells in their undifferentiated state; thereby inhibiting differentiation and expanding said hematopoietic cells *ex-vivo;* and
(b) transducing said therapeutic expanded, undifferentiated hematopoietic stem and progenitor cells with the exogene, with the proviso that said hematopoietic stem and progenitor cells are not human embryonic stem cells obtained from a human embryo.

14. The method of claim 13, wherein said transducing is effected by a vector including the exogene.

15. A therapeutic ex vivo cultured hematopoietic stem and progenitor cell population comprising undifferentiated hematopoietic stem and progenitor cells expanded according to the methods of any of claims 1-14, said population having:
(i) a greater proportion of proliferating hematopoietic stem and progenitor cells;
(ii) a greater proportion of clonogenic cells (CFUc); and
(iii) a greater proportion of undifferentiated cells maintained in their undifferentiated state
as compared to a similar population of hematopoietic stem and progenitor cells ex-vivo cultured without contacting said transition metal chelator with the proviso that said hematopoietic cells are not human embryonic stem cells obtained from a human embryo.

16. A method of preservation of undifferentiated hematopoietic stem and progenitor cells comprising contacting the undifferentiated hematopoietic stem and progenitor cells with a transition metal chelator selected from the group consisting of TEPA, EDA, PEHA, TETA, penicilamine and captopril, wherein said contacting is performed in at least one of the steps of harvesting, isolating and storage of the undifferentiated hematopoietic cells, with the proviso that said hematopoietic cells are not human embryonic stem cells obtained from a human embryo.

17. The method of claim 16, wherein said transition metal chelator is TEPA.

18. Stem cell collection bags, supplemented with an amount of a transition metal chelator selected from the group consisting of TEPA, EDA, PEHA, TETA, penicilamine and captopril, said amount sufficient to inhibit differentiation of a population of undifferentiated hematopoietic cells.

19. The stem cell collection bags of claim 18, wherein said transition metal chelator is TEPA.

20. The stem cell collection bags of claim 18, wherein said transition metal chelator concentration is 0.1 µM to 100 mM.

21. The stem cell collection bags of claim 20, wherein said transition metal chelator concentration is 4 µM to 50 mM.

22. The stem cell collection bags of claim 20, wherein said transition metal chelator concentration is 5µM to 40 mM.

23. The use of a therapeutic hematopoietic cell composition comprising a population of undifferentiated hematopoietic stem and progenitor cells expanded according to the methods of any of claims 1-14 for the preparation of a medicament for treating a subject in need of hematopoietic stem cell transplantation, wherein said population is **characterized by** having:
(i) a greater proportion of proliferating hematopoietic stem and progenitor cells;
(ii) a greater proportion of clonogenic cells (CFUc); and
(iii) a greater proportion of undifferentiated cells maintained in their undifferentiated state
as compared to a similar population of hematopoietic stem and progenitor cells ex-vivo cultured without contacting said transition metal chelator.

24. The methods of claims 1 and 13, wherein said at least an early acting cytokine or cytokines are selected from the group consisting of stem cell factor, FLT3 ligand, interleukin-6, thrombopoietin and interleukin-3.

25. The methods of claims 1 and 13, wherein said early acting cytokine is FLT3 ligand.

26. A therapeutic ex vivo cultured hematopoietic stem and progenitor cell population comprising undifferentiated hematopoietic stem and progenitor cells expanded according to the methods of any of claims 1-14, wherein said cell population is provided in a medium comprising said transition metal chelator with the proviso that said hematopoietic cells are not human embryonic stem cells obtained from a human embryo.

## Patentansprüche

1. Verfahren zum ex-vivo-Anreichern von hämatopoetischen Stamm- und Vorläuferzellen, während zur gleichen Zeit die Differenzierung der hämatopoetischen Stamm- und Vorläuferzellen inhibiert wird, wobei das Verfahren umfasst: das Bereitstellen der hämatopoetischen Stamm- und Vorläuferzellen ex-vivo mit Bedingungen zur Zellproliferation und mit einem Übergangsmetall-Chelator ausgewählt aus der Gruppe bestehend aus Tetraethylenpentamin (TEPA), Ethylendiamin (EDA), Pentaethylenhexamin (PEHA), Triethylentetramin (TETA), Penicillamin und Captopril, wobei die Bedingungen zur Zellproliferation Nährstoffe und mindestens ein früh agierendes Cytokin oder früh agierende Cytokine umfassen, wobei die hämatopoetischen Stamm- und Vorläuferzellen therapeutische hämatopoetische Stamm- und Vorläuferzellen sind und wobei der Chelator und die proliferativen Bedingungen resultieren in
(i) verlängerter aktiver Proliferation;
(ii) langzeitiger Expansion von clonogenen Zellen (CFUc); und
(iii) Erhalten von undifferenzierten Zellen in deren undifferenziertem Zustand; mit der Maßgabe, dass die hämatopoetischen Zellen keine menschlichen embryonalen Stammzellen sind, die von einem menschlichen Embryo erhalten werden.

2. Verfahren nach Anspruch 1, wobei der Übergangsmetall-Chelator TEPA ist.

3. Verfahren nach Anspruch 1, wobei die Konzentration des Übergangsmetall-Chelators 0,1 µM bis 100 mM ist.

4. Verfahren nach Anspruch 3, wobei die Konzentration des Übergangsmetall-Chelators 4 µM bis 50 mM ist.

5. Verfahren nach Anspruch 4, wobei die Konzentration des Übergangsmetall-Chelators 5 µM bis 40 mM ist.

6. Verfahren nach Anspruch 1, wobei die Bedingungen zur Zellproliferation des Weiteren das Bereitstellen eines spät agierenden Cytokins oder spät agierender Cytokine umfassen.

7. Verfahren nach Anspruch 6, wobei das spät agierende Cytokin ausgewählt ist aus der Gruppe bestehend aus Granulocyten-Kolonie-stimulierendem Faktor, Granulocyten/Makrophagen-Kolonie-stimulierendem Faktor und Erythropoietin.

8. Verfahren nach Anspruch 6, wobei das spät agierende Cytokin Granulocyten-Kolonie-stimulierender Faktor ist.

9. Verfahren nach Anspruch 1, wobei die hämatopoetischen Zellen von einer Quelle stammen, ausgewählt aus der Gruppe bestehend aus Knochenmarkzellen, peripheren Blutzellen und neonatalen Nabelschnurblutzellen.

10. Verfahren nach Anspruch 1, wobei die hämatopoetischen Stamm- und Vorläuferzellen von neonatalen Nabelschnurblutzellen abstammen.

11. Verfahren nach Anspruch 1, des Weiteren umfassend den Schritt des Auswählens einer Population von hämatopoetischen Zellen, die angereichert sind mit hämatopoetischen Stammzellen.

12. Verfahren nach Anspruch 11, wobei die Zellen mit CD34+-Zellen angereichert sind.

13. Verfahren zum Transduzieren von expandierten, undifferenzierten therapeutischen hämatopoetischen Stamm- und Vorläuferzellen mit einem Exogen, wobei das Verfahren umfasst:
(a) Expandieren und Inhibieren der Differenzierung der hämatopoetischen Stamm- und Vorläuferzellen durch:
(i) Bereitstellen der hämatopoetischen Stamm- und Vorläuferzellen mit Bedingungen zur Zellproliferation, wobei die Bedingungen zur Zellproliferation Nährstoffe und mindestens ein früh agierendes Cytokin oder früh agierende Cytokine umfassen und wobei die hämatopoetischen Stamm- und Vorläuferzellen therapeutische hämatopoetische Stamm- und Vorläuferzellen sind;
(ii) Inkontaktbringen der hämatopoetischen Stamm- und Vorläuferzellen mit einem Übergangsmetall-Chelator ausgewählt aus der Gruppe bestehend aus TEPA, EDA, PEHA, TETA, Penicillamin und Captopril, wobei der Chelator und die Proliferationsbedingungen zu verlängerter, aktiver Proliferation; Langzeit-Expansion von clonogenen Zellen (CFUc); und Erhalten von undifferenzierten Zellen in deren undifferenziertem Zustand führen; wodurch die Differenzierung und Expansion der hämatopoetischen Zellen *ex-vivo* inhibiert wird; und
(b) Transduzieren der therapeutischen expandierten, undifferenzierten hämatopoetischen Stamm- und Vorläuferzellen mit dem Exogen, mit der Maßgabe, dass die hämatopoetischen Stamm- und Vorläuferzellen keine menschlichen embryonalen Stammzellen sind, die von einem menschlichen Embryo erhalten werden.

14. Verfahren nach Anspruch 13, wobei das Transduzieren mit einem Vektor, der das Exogen enthält, durchgeführt wird.

15. Therapeutische, ex-vivo-gezüchtete hämatopoetische Stamm- und Vorläufer-Zellpopulation, umfassend undifferenzierte hämatopoetische Stamm- und Vorläuferzellen, expandiert gemäß dem Verfahren nach einem der Ansprüche 1 bis 14, wobei die Population:
(i) einen größeren Anteil an proliferativen hämatopoetischen Stamm- und Vorläuferzellen;
(ii) einen größeren Anteil an clonogenen Zellen (CFUc); und
(iii) einen größeren Anteil an undifferenzierten Zellen, die in deren undifferenziertem Zustand erhalten werden,
hat, im Vergleich zu einer gleichen Population von hämatopoetischen Stamm- und Vorläuferzellen, die *ex-vivo* gezüchtet werden ohne Inkontaktbringen mit dem Übergangsmetall-Chelator, mit der Maßgabe, dass die hämatopoetischen Zellen keine menschlichen embryonalen Stammzellen sind, die von einem menschlichen Embryo erhalten werden.

16. Verfahren zur Bewahrung von undifferenzierten hämatopoetischen Stamm- und Vorläuferzellen umfassend das Inkontaktbringen der undifferenzierten hämatopoetischen Stamm- und Vorläuferzellen mit einem Übergangsmetall-Chelator ausgewählt aus der Gruppe bestehend aus TEPA, EDA, PEHA, TETA, Penicillamin und Captopril, wobei das Inkontaktbringen mindestens bei einem der Schritte des Erntens, Isolierens und Lagerns der undifferenzierten hämatopoetischen Zellen erfolgt, mit der Maßgabe, dass die hämatopoetischen Zellen keine menschlichen embryonalen Stammzellen sind, die von einem menschlichen Embryo erhalten werden.

17. Verfahren nach Anspruch 16, wobei der Übergangsmetall-Chelator TEPA ist.

18. Stammzellensammelbeutel, ergänzt mit einer Menge an Übergangsmetall-Chelator ausgewählt aus der Gruppe bestehend aus TEPA, EDA, PEHA, TETA, Penicillamin und Captopril, wobei die Menge ausreichend ist, um die Differenzierung einer Population von undifferenzierten hämatopoetischen Zellen zu inhibieren.

19. Stammzellensammelbeutel nach Anspruch 18, wobei der Übergangsmetall-Chelator TEPA ist.

20. Stammzellensammelbeutel nach Anspruch 18, wobei die Konzentration des Übergangsmetall-Chelators 0,1 µM bis 100 mM ist.

21. Stammzellensammelbeutel nach Anspruch 20, wobei die Konzentration des Übergangsmetall-Chelators 4 µM bis 50 mM ist.

22. Stammzellensammelbeutel nach Anspruch 20, wobei die Konzentration des Übergangsmetall-Chelators 5 µM bis 40 mM ist.

23. Verwendung einer therapeutischen hämatopoetischen Zellzusammensetzung umfassend eine Population von undifferenzierten hämatopoetischen Stamm- und Vorläuferzellen expandiert gemäß den Verfahren nach einem der Ansprüche 1 bis 14 für die Herstellung eines Arzneimittels zur Behandlung eines Individuums, das eine hämatopoetische Stammzelltransplantation benötigt, wobei die Population **dadurch gekennzeichnet ist, dass** sie:
(i) einen größeren Anteil an proliferierenden hämatopoetischen Stamm- und Vorläuferzellen;
(ii) einen größeren Anteil an clonogenen Zellen (CFUc); und
(iii) einen größeren Anteil an undifferenzierten Zellen, die in deren undifferenziertem Zustand erhalten werden,
hat, im Vergleich zu einer gleichen Population von hämatopoetischen Stamm- und Vorläuferzellen, die *ex-vivo* ohne Inkontaktbringen mit dem Übergangsmetall-Chelator gezüchtet wurden.

24. Verfahren nach Anspruch 1 und 13, wobei mindestens (ein) früh agierende(s) Cytokin oder Cytokine ausgewählt sind aus der Gruppe bestehend aus Stammzellfaktor, FLT3-Ligand, Interleukin-6, Thrombopoietin und Interleukin-3.

25. Verfahren nach Anspruch 1 und 13, wobei das früh agierende Cytokin ein FLT3-Ligand ist.

26. Therapeutische, ex-vivo-gezüchtete hämatopoetische Stamm- und Vorläuferzellenpopulation umfassend undifferenzierte hämatopoetische Stamm- und Vorläuferzellen expandiert gemäß den Verfahren nach einem der Ansprüche 1 bis 14, wobei die Zellpopulation in einem Medium umfassend den Übergangsmetall-Chelator bereit gestellt wird, mit der Maßgabe, dass die hämatopoetischen Zellen keine menschlichen embryonalen Stammzellen sind, die von einem menschlichen Embryo erhalten werden.

## Revendications

1. Procédé d'expansion de cellules souches et progénitrices hématopoïétiques ex-vivo, inhibant en même temps la différenciation desdites cellules souches et progénitrices hématopoïétiques, le procédé comprenant la fourniture de conditions de prolifération cellulaire pour lesdites cellules souches et progénitrices hématopoïétiques *ex vivo* avec un chélateur de métal de transition choisi dans le groupe constitué par la tétraéthylène pentamine (TEPA), l'éthylène diamine (EDA), la pentaéthylène hexamine (PEHA), la triéthylène tétramine (TETA), la pénicilamine et le captopril, dans lequel lesdites conditions de prolifération cellulaire comprennent des nutriments et au moins une cytokine à action précoce ou des cytokines à action précoce, dans lequel lesdites cellules souches et progénitrices hématopoïétiques sont des cellules souches et progénitrices hématopoïétiques thérapeutiques, et dans lequel ledit chélateur et lesdites conditions de prolifération résultent en
(i) une prolifération active prolongée ;
(ii) une expansion à long terme de cellules clonogènes (CFUc) ; et
(iii) le maintien des cellules indifférenciées dans leur état indifférencié ;
à condition que lesdites cellules hématopoïétiques ne soient pas des cellules souches embryonnaires humaines obtenues à partir d'un embryon humain.

2. Procédé selon la revendication 1, dans lequel ledit chélateur de métal de transition est TEPA.

3. Procédé selon la revendication 1, dans lequel la concentration dudit chélateur de métal de transition est de 0,1 µM à 100 mM.

4. Procédé selon la revendication 3, dans lequel la concentration dudit chélateur de métal de transition est de 4 µM à 50 mM.

5. Procédé selon la revendication 4, dans lequel la concentration dudit chélateur de métal de transition est de 5 µM à 40 mM.

6. Procédé selon la revendication 1, dans lequel lesdites conditions de prolifération cellulaire comprennent, en outre, la fourniture d'une cytokine à action tardive ou de cytokines à action tardive.

7. Procédé selon la revendication 6, dans lequel ladite cytokine à action tardive est choisie dans le groupe constitué par le facteur de stimulation des colonies granulocytaires, le facteur de stimulation des colonies granulocytaires/macrophages et l'érythropoïétine.

8. Procédé selon la revendication 6, dans lequel ladite cytokine à action tardive est le facteur de stimulation des colonies granulocytaires.

9. Procédé selon la revendication 1, dans lequel lesdites cellules hématopoïétiques sont dérivées d'une source choisie dans le groupe constitué par les cellules de moelle osseuse, les cellules du sang périphérique et les cellules du sang de cordon ombilical néonatal.

10. Procédé selon la revendication 1, dans lequel lesdites cellules souches et progénitrices hématopoïétiques sont dérivées de cellules du sang de cordon ombilical néonatal.

11. Procédé selon la revendication 1, comprenant, en outre, l'étape consistant à sélectionner une population de cellules hématopoïétiques enrichie en cellules souches hématopoïétiques.

12. Procédé selon la revendication 11, dans lequel lesdites cellules sont enrichies en CD34+.

13. Procédé de transduction de cellules souches et progénitrices hématopoïétiques thérapeutiques indifférenciées, expansées avec un exogène, le procédé comprenant les étapes consistant à :
(a) expanser et inhiber la différenciation desdites cellules souches et progénitrices hématopoïétiques par :
(i) fourniture de conditions de prolifération cellulaire pour lesdites cellules souches et progénitrices hématopoïétiques, dans lequel lesdites conditions de prolifération cellulaire comprennent des nutriments et au moins une cytokine à action précoce ou des cytokines à action précoce et dans lequel lesdites cellules souches et progénitrices hématopoïétiques sont des cellules souches et progénitrices hématopoïétiques thérapeutiques ;
(ii) mise en contact desdites cellules souches et progénitrices hématopoïétiques avec un chélateur de métal de transition choisi dans le groupe constitué par TEPA, EDA, PEHA, TETA, la pénicilamine et le captopril, dans lequel ledit chélateur et lesdites conditions de prolifération induisent une prolifération active prolongée ; une expansion à long terme de cellules clonogènes (CFUc) ; et le maintien des cellules indifférenciées à leur état indifférencié ; pour inhiber ainsi la différenciation et expanser lesdites cellules hématopoïétiques *ex-vivo* ; et
(b) transduire lesdites cellules souches et progénitrices hématopoïétiques thérapeutiques expansées et indifférenciées avec l'exogène, à condition que lesdites cellules souches et progénitrices hématopoïétiques ne soient pas des cellules souches embryonnaires humaines obtenues à partir d'un embryon humain.

14. Procédé selon la revendication 13, dans lequel ladite transduction est réalisée par un vecteur contenant l'exogène.

15. Population de cellules souches et progénitrices hématopoïétiques thérapeutiques cultivée *ex-vivo* comprenant des cellules souches et progénitrices hématopoïétiques indifférenciées, expansées selon les procédés de l'une quelconque des revendications 1 à 14, ladite population ayant :
(i) une plus grande proportion de cellules souches et progénitrices hématopoïétiques en cours de prolifération ;
(ii) une plus grande proportion de cellules clonogènes (CFUc) ; et
(iii) une plus grande proportion de cellules indifférenciées maintenues à leur état indifférencié
comparativement à une population similaire de cellules souches et progénitrices hématopoïétiques cultivée *ex-vivo* sans mise en contact avec ledit chélateur de métal de transition, à condition que lesdites cellules hématopoïétiques ne soient pas des cellules souches embryonnaires humaines obtenues à partir d'un embryon humain.

16. Procédé de conservation de cellules souches et progénitrices hématopoïétiques indifférenciées comprenant la mise en contact des cellules souches et progénitrices hématopoïétiques indifférenciées avec un chélateur de métal de transition choisi dans le groupe constitué par TEPA, EDA, PEHA, TETA, la pénicilamine et le captopril, dans lequel ladite mise en contact s'effectue au moins au cours d'une des étapes de récolte, isolation et stockage des cellules hématopoïétiques indifférenciées, à condition que lesdites cellules hématopoïétiques ne soient pas des cellules souches embryonnaires humaines obtenues à partir d'un embryon humain.

17. Procédé selon la revendication 16, dans lequel ledit chélateur de métal de transition est TEPA.

18. Sacs de collecte de cellules souches, additionnés d'une quantité de chélateur de métal de transition choisi dans le groupe constitué par TEPA, EDA, PEHA, TETA, la pénicilamine et le captopril, ladite quantité étant suffisante pour inhiber la différenciation d'une population de cellules hématopoïétiques indifférenciées.

19. Sacs de collecte de cellules souches selon la revendication 18, dans lesquels ledit chélateur de métal de transition est TEPA.

20. Sacs de collecte de cellules souches selon la revendication 18, dans lesquels la concentration dudit chélateur de métal de transition est de 0,1 µM à 100 mM.

21. Sacs de collecte de cellules souches selon la revendication 20, dans lesquels la concentration dudit chélateur de métal de transition est de 4 µM à 50 mM.

22. Sacs de collecte de cellules souches selon la revendication 20, dans lesquels la concentration dudit chélateur de métal de transition est de 5 µM à 40 mM.

23. Utilisation d'une composition de cellules hématopoïétiques thérapeutiques comprenant une population de cellules souches et progénitrices hématopoïétiques indifférenciées, expansées selon les procédés de l'une quelconque des revendications 1 à 14, dans la préparation d'un médicament destiné à traiter un sujet ayant besoin d'une greffe de cellules souches hématopoïétiques, dans laquelle ladite population est **caractérisée en ce qu'**elle a :
(i) une plus grande proportion de cellules souches et progénitrices hématopoïétiques en cours de prolifération ;
(ii) une plus grande proportion de cellules clonogènes (CFUc) ; et
(iii) une plus grande proportion de cellules indifférenciées maintenues à leur état indifférencié
comparativement à une population similaire de cellules souches et progénitrices hématopoïétiques cultivée *ex-vivo* sans mise en contact avec ledit chélateur de métal de transition.

24. Procédés selon les revendications 1 et 13, dans lesquels ladite au moins ou lesdites cytokines à action précoce sont choisies dans le groupe constitué par le facteur de cellule souche, le ligand FLT3, l'interleukine-6, la thrombopoïétine et l'interleukine-3.

25. Procédés selon les revendications 1 et 13, dans lesquels ladite cytokine à action précoce est le ligand FLT3.

26. Population de cellules souches et progénitrices hématopoïétiques thérapeutiques cultivée *ex-vivo* comprenant des cellules souches et progénitrices hématopoïétiques indifférenciées, expansées selon les procédés de l'une quelconque des revendications 1 à 14, dans laquelle ladite population de cellules est fournie dans un milieu comprenant ledit chélateur de métal de transition, à condition que lesdites cellules hématopoïétiques ne soient pas des cellules souches embryonnaires humaines obtenues à partir d'un embryon humain.
